# EUROPÄISCHE PATENTANMELDUNG

(11) **EP 2 679 302 A1**
(43) Veröffentlichungstag der Anmeldung: **01.01.2014**
(21) Anmeldenummer: 12174028.6
(22) Anmeldetag: 28.06.2012
(51) Int. Cl.: B01J 20/28, B01J 20/32, B01J 20/26, A61K 31/745, A61M 1/36

(54) **Selektives Sorptionsmittel für die extrakorporale Blutreinigung**

(71) Anmelder: Zentrum für biomedizinische Technologie der Donau- Universität Krems, 3500 Krems (AT)
(72) Erfinder: Falkenhagen, Dieter, 3500 Krems (AT); Hartmann, Jens, 3511 Furth (AT); Harm, Stephan, 3508 Krustetten (AT)
(74) Vertreter: Patentanwaltskanzlei Matschnig & Forsthuber OG

(57) **Zusammenfassung**

Die Erfindung betrifft ein Sorptionsmittel umfassend eine poröse Sorptionsmatrix mit einer neutralen, hydrophoben Oberfläche, einer mittleren Porengröße von 11 bis 20 nm und einer mittleren Partikelgröße von 75 bis 150 µm, zur Behandlung von Erkrankungen und Zuständen, die mit einer erhöhten Konzentration wenigstens eines Entzündungsmediators, insbesondere Zytokins, assoziiert sind, insbesondere zur Behandlung einer Sepsis. Die Erfindung betrifft ferner eine Sorptionseinrichtung (107, 207, 307), enthaltend wenigstens ein derartiges Sorptionsmittel. Die Erfindung betrifft auch eine extrakorporale Perfusionsvorrichtung (100, 200, 300), umfassend wenigstens ein derartiges Sorptionsmittel bzw. wenigstens eine derartige Sorptionseinrichtung (107, 207, 307).

## Beschreibung

Die Erfindung betrifft ein Sorptionsmittel umfassend eine poröse Sorptionsmatrix mit einer neutralen, hydrophoben Oberfläche. Das Sorptionsmittel weist signifikant verbesserte selektive Eigenschaften in Bezug auf das Entfernen oder Abreichern von Entzündungsmediatoren aus Körperflüssigkeiten auf und ist daher besonders vorteilhaft in der Behandlung von Erkrankungen und Zuständen, die mit einer erhöhten Konzentration wenigstens eines Entzündungsmediators, insbesondere aus der Gruppe der Zytokine, vorzugsweise des Schlüsselzytokins TNF-alpha assoziiert sind, insbesondere zur Behandlung einer Sepsis und Sepsis-ähnlichen Zuständen.

Sepsis und damit verbundenen Komplikationen tragen in einem nicht unbeachtlichen Ausmaß zur Morbidität und Mortalität bei Menschen bei. In den meisten Fällen ist eine Sepsis auf eine Infektion mit gram-negativen Bakterien zurückzuführen, wenn hohe Endotoxinkonzentrationen in den Körper gelangen und systemische Wirkung zeigen. Endotoxine sind Lipopolysaccharide (LPS) in der Zellwand gramnegativer Bakterien und werden durch Zelllyse freigesetzt. Eine Sepsis ist unter anderem durch hohes Fieber, niedrigen Blutdruck und, in schweren Fällen wie dem septischen Schock, durch Multiorganversagen gekennzeichnet. Eine Sepsis ist eine sehr ernstzunehmende Erkrankung; die Letalität von Individuen mit schwerer Sepsis oder septischem Schock liegt je nach Schweregrad der Erkrankung bei ca. 30-60%. Auch Patienten mit beeinträchtigter Immunabwehr, wie z.B. Leberpatienten oder Patienten in Chemotherapie, neigen zu bakteriellen Infektionen und zeigen dadurch Symptome einer Endotoxinvergiftung. Bei akutem Leberversagen oder einer akuten Dekompensation bei chronischem Leberversagen ergeben sich Zustände, die - biochemisch gesehen - einer Sepsis sehr ähnlich sind. Beispielsweise kann es bei Patienten mit chronischem Leberversagen zu einer akuten Dekompensation kommen. In diesem Zustand können Endotoxine der normalen Darmflora die Darmbarriere überwinden und im Körper die Freisetzung von Entzündungsmediatoren stimulieren und somit einen Sepsis ähnlichen Zustand hervorrufen.

Ferner können septische Zustände auch durch gram-positive Bakterien, Viren und Pilze ausgelöst werden.

Es ist allgemein bekannt, dass es bei einer Sepsis und bei anderen schwerwiegende Erkrankungen zu einer unkontrollierten Aktivierung der Immunzellen und zu einem Ungleichgewicht des Gerinnungssystems kommen kann. Die unkontrollierte Aktivierung des mononukleär-phagozytären Systems stimuliert eine übermäßige Freisetzung von Entzündungsmediatoren, insbesondere von Zytokinen (auch als Zytokinsturm oder Hyperzytokinämie bezeichnet). Zytokine gelten als Schlüsselmediatoren bei Sepsis und septischem Schock. Als wichtigste pro-inflammatorische Vertreter sind Tumor-Nekrose-Faktor (TNF-α, oft auch nur als TNF bezeichnet) und Interleukin-1β (IL-1β) zu nennen. Weitere wichtige pro-inflammatorische Zytokine sind IL-6 und IL-8. Das initial freigesetzte Zytokin TNF-α löst über eine Mediatorkaskade eine biologischen Signalverstärkung aus, die zu physiologischen Veränderungen, einschließlich schwerer Störungen des biologischen Gleichgewichts, und in weiterer Folge zu Kreislaufkollaps und Multiorganversagen führt. Das klinische Bild einer Sepsis korreliert mit hohen Blut-Konzentrationen des Schlüsselmediators TNF-α aber auch anderer Zytokine wie IL1-ß, IL-6, IL-8 im Falle der proinflammatorischen Phase bzw. IL-10 bzw. IL-13 bei Auftreten einer anti-inflammatorischen Phase, in der die proinflammatorischen Mediatoren inklusive Zytokine sehr niedrige Konzentrationen aufweisen. Ferner stehen auch andere schwerwiegende Erkrankungen wie chronisch-entzündliche Darmerkrankungen, Psoriasis und Rheumatoide Arthritis mit einer übermäßigen TNF-α-Freisetzung im Zusammenhang.

Zur Behandlung einer Sepsis werden neben der standardmäßig angewandten intensivmedizinischen Behandlung vor allem Antibiotika bzw. Corticosteroide, Immunglobuline sowie Kreislauf-unterstützende Arzneimittel eingesetzt.

Ein Nachteil der Antibiotika-Therapie ist die zunehmende Verbreitung antibiotikaresistenter Bakterien. Ferner werden durch das Antibiotikum und der damit einhergehenden Zerstörung der Bakterienzellen Endotoxine vermehrt freigesetzt, was wiederum zu einer verstärkten Ausschüttung von Entzündungsmediatoren führt. Zudem ist eine Antibiotikagabe häufig mit Nebenwirkungen wie Veränderungen der Darmflora oder allergischen Reaktionen verbunden.

Der Versuch, Antikörper gegen den Schlüsselfaktor TNF-alpha einzusetzen, scheiterte, da offenbar bei dieser Methode die Absenkung der TNF-Konzentration auf Null oder sehr geringe Werte eine anergische Situation auslöste, die von einer höheren Mortalität im Vergleich zur Kontrollgruppe begleitet war. Der therapeutische Einsatz spezifischer Antikörper gegen LPS und TNF-α ist technisch sehr aufwändig und daher mit hohen Kosten verbunden. Mittels extrakorporaler Blutreinigungssysteme wird wie im Folgenden näher beschrieben daher versucht, die genannten Zytokine insbesondere den Faktor TNF-α in einer die Konzentrationen dieser Zytokine normalisierenden Weise zu entfernen, um somit die anergische (antiinflammatorische) Phase zu umgehen. Mittels sogenannter LPS-Adsorber (z.B. der Adsorber Toraymyxin®) werden die Endotoxine eliminiert, um somit eine Freisetzung der proinflammatorischen Zytokine zu vermeiden, die natürlich auch die antiinflammatorische Reaktion mindert.

Alternativ zu den oben genannten medikamentösen Therapieansätzen können toxische Blutbestandteile mit Hilfe von extrakorporalen Blut- bzw. Plasmareinigungsverfahren (therapeutische Aphereseverfahren) unter Verwendung geeigneter Sorptionsmittel (Adsorber) aus dem Blut bzw. Blutplasma eliminiert werden. Bei der therapeutischen Apherese können pathophysiologisch relevante Blut- und Plasmakomponenten, beispielsweise Biomoleküle wie (Glyko)Proteine, Peptide, Lipide, Lipoproteine und Lipopolysaccharide sowie Toxine, aber auch Blutzellen und Blutplasma auf spezifische Art und Weise entfernt werden. Aphereseverfahren können einerseits zu diagnostischen und therapeutischen Zwecken eingesetzt werden, andererseits stellen sie auch eine sehr effektive Möglichkeit dar, bestimmte Blutbestandteile von gesunden Individuen in ausreichender Menge und in ausreichend hoher Reinheit zu gewinnen. Große Bedeutung wird der therapeutischen Apherese zugemessen, da diese bei bestimmten Indikationen oft eine sehr wirkungsvolle und gleichzeitig nebenwirkungsarme Alternative zur medikamentösen Behandlung ist. So kann bei Plasmaphereseverfahren das Plasma entweder vollständig abgetrennt und durch eine Substitutionslösung ersetzt werden, oder es werden nur bestimmte Bestandteile wie LDL, Endotoxine, oder Immunglobuline mittels eines geeigneten Sorptionsmittels daraus entfernt und das Plasma anschließend in den Spender/Patienten wieder zurückgeführt. Therapeutische Aphereseverfahren haben gegenüber den genannten medikamentösen Behandlungsstrategien auch den Vorteil, dass die Behandlung jederzeit durch Abschalten der Apheresevorrichtung mit sofortiger Wirkung angehalten wird.

Aphereseverfahren und Adsorbermaterialien zum Eliminieren toxischer und/oder schädlicher Blutbestandteile sind im Stand der Technik gut bekannt. Ebenso bekannt sind Adsorbermaterialien, welche spezifisch Zytokine, insbesondere TNF-α, adsorbieren und aus Körperflüssigkeiten wie Blut oder Blutplasma entfernen. Das Dokument US 2001/0070424 A1 offenbart ein Adsorbermaterial basierend auf einem porösen Polymer, welches zumindest eine Transportpore mit einem Durchmesser von 25 bis 200 nm sowie effektive Poren mit einem Durchmesser von 10 bis 25 nm aufweist. Das Polymer kann unter anderem auch ein nicht-ionisches Harz (Neutralharz) sein. Der Adsorber wird zum Entfernen von Proteinmolekülen, insbesondere Zytokinen und β2-Mikroglobulin, verwendet.

Das Dokument WO 2011/123767 A1 offenbart ein Verfahren zum Behandeln von Entzündungen, wobei einem Patienten eine therapeutisch wirksame Dosis an porösen Adsorberpartikeln zum Adsorbieren von Entzündungsmediatoren verabreicht wird, wobei das Gesamtporenvolumen bei einer Porengröße von 5 bis 300 nm größer als 0,5 cc/g bis 3,0 cc/g ist.

In der WO 2003/090924 ist eine poröse Abtrennmatrix zum Abtrennen von Blutbestandteilen im Zusammenhang mit Entzündungsprozessen beschrieben. Die Abtrennmatrix weist eine Porengröße von 5 µm bis 500 µm sowie wenigstens eine an der Matrix angeordnete funktionelle Gruppe auf.

Die DE 19515554 A1 offenbart Verfahren und Vorrichtungen zur simultanen extrakorporalen Elimination von TNF-α und Lipopolysacchariden aus Vollblut und/oder Blutplasma. Dabei wird das Blut oder Blutplasma in einem extrakorporalen Perfusionssystem über ein poröses Kationenaustauscher- und ein Anionenaustauschermaterial geleitet. Die darin beschriebenen porösen Trägermaterialien weisen einen mittleren Porendurchmesser von < 30 nm und/oder eine molekulare Ausschlussgröße für globuläre Proteine von < 10⁶ Dalton und insbesondere < 2 x 10⁴ Dalton auf.

Neutralharze zum Entfernen toxischer Bestandteile, einschließlich Zytokine, aus einer Körperflüssigkeit sind weiters in der WO 2005/082504 A2 offenbart. Die WO 2005/082504 A2 beschreibt eine Entgiftungsvorrichtung, welche Aktivkohle und zumindest ein nicht-ionisches Harz mit einer mittleren Porengröße von 30 nm und einem mittleren Durchmesser von 35-120 µm (Amberchrom CG300C) bzw. mit einer mittleren Porengröße von 45 nm und einem mittleren Durchmesser von 560 µm (Harz auf Basis aliphatischer Ester - Amberlite XAD-7HP) aufweist.

EP 0787500 B1 und EP 0958839 B1 offenbaren ein hydrophobes Trägermaterial mit einer Porengröße von 10 bis 30 nm und Partikelgrößen von 20 bis 350 µm, vorzugsweise 10 bis 100 µm oder 250 bis 350 µm zum Entfernen von toxischen Bestandteilen, insbesondere Zytokinen aus einer Körperflüssigkeit.

Die EP 1 944 046 B1 offenbart ein Trägermaterial auf Basis eines Polystyrol-Divinylbenzol-Copolymers mit einer Porengröße von 30 nm und einer Partikelgröße von 75 bis 120 µm.

Tetta et al. (Tetta et al., Nephrol Dial Transplant (1998) 13:1458-1464) beschreiben einen Adsorber des Amberchrom-Typs CG 300md mit einer Porengröße von 30 nm zum Entfernen von Zytokinen aus einer Körperflüssigkeit.

Die Veröffentlichung von Cantaluppi et al. (Cantaluppi et al., Critical Care (2010) 14:R4) beschreibt einen Adsorber des Typs Amberchrom CG161m zur Zytokinadsorption.

Die oben genannten Adsorber haben den Nachteil einer geringen Effektivität und/oder Selektivität, so dass neben den toxischen/schädlichen Blutbestandteilen wie Lipopolysacchariden und Entzündungsmediatoren auch physiologisch wichtige Proteine bzw. Faktoren des Blutgerinnungssytems (Antikoagulanzien sowie Prokoagulanzien) sehr effektiv eliminiert werden. Hier sind insbesondere wichtige patienteneigene Gerinnungsfaktoren wie Protein C und Protein S oder Fibrinogen zu nennen. Von besonderer Wichtigkeit ist das Protein C als gerinnungshemmender Faktor. Eine Verminderung der intrakorporalen Protein C-Konzentration führt zu schweren Gerinnungskomplikationen wie Venenthrombosen und Lungenembolien. Auch das bei extrakorporalen Blutreinigungsverfahren häufig eingesetzte und per Infusion zusätzlich in den extrakorporalen Blutkreislauf zugeführte Antikoagulans Heparin wird durch Anionenaustauscher besonders rasch eliminiert. Die Folge ist ein Ungleichgewicht des intrakorporalen Gerinnungssystems bzw. eine unerwünschte Antikoagulation im extrakorporalen bzw. intrakorporalen Blutkreislauf. Dies kann besonders für Hochrisikopatienten wie Patienten mit Leberversagen und Sepsis sehr problematisch sein. Über das große Risiko einer okklusiven Thrombosebildung im Rücklauf des extrakorporalen Blutreinigungssystems bzw. im Patienten nach Rückführung des gereinigten Blutes durch die unerwünschte Bindung von Proteinen des Blutgerinnungssystems am Anionenaustauscher wurde kürzlich berichtet [Meijers et al. 2007. Major Coagulation Disturbances During Fractionated Plasma Separation and Adsorption. Am J Transplant 7(9):2195-2199].

In der DE 199 13 707 A1 wird ein Immunadsorber zum Einsatz in der Sepsistherapie für die Plasmapherese beschrieben, bestehend aus einem Trägermaterial aus organischen oder synthetischen Polymeren und darauf gebundenen poly- oder monoklonalen Antikörpern, die gegen Komplementfaktoren, Lipopolysaccharide sowie gegen weitere Sepsis-Mediatoren wie z.B. TNF-α und Interleukine gerichtet sind.

Die DE 10 2004 029 573 A1 offenbar ein Apheresematerial bzw. Adsorbens sowie ein Verfahren für die Entfernung, Abreicherung oder Inaktivierung des Zytokins MIF (Makrophagenmigrationsinhibierender Faktor) aus Blut, Blutplasma oder anderen Körperflüssigkeiten. Das Adsorbens umfasst ein festes Trägermaterial auf dessen Oberfläche MIF-bindende Moleküle oder funktionale Gruppen immobilisiert sind.

In der DE 10 2005 046 258 A1 ist ein Immunadsorber zur Behandlung von Insulinresistenz und/oder des metabolischen Syndroms geoffenbart, wobei der Immunadsorber Trägermaterialen mit gebundenen Liganden, die für IL-6, IL-4 und C5a spezifisch sind, umfasst.

Die weiter oben beschriebene Gerinnungsproblematik kann durch die Verwendung solcher spezifischen Immunadsorber umgangen werden. Allerdings ist die Herstellung solcher Adsorber technisch äußerst aufwändig und aufgrund der hohen Kosten aus ökonomischen Gründen nur limitiert anwendbar.

Es ist eine Aufgabe der Erfindung, die aus dem Stand der Technik bekannten Nachteile zu beseitigen bzw. zu minimieren und ein Sorptionsmittel mit verbesserten Selektivitätseigenschaften und höherer Biokompatibilität bereitzustellen. Folglich ist es eine Aufgabe der Erfindung, ein verbessertes Sorptionsmittel bereitzustellen, mit welchem toxische Bestandteile wie Entzündungsmediatoren, insbesondere Zytokine, aus einer Körperflüssigkeit hocheffektiv abgetrennt werden, während an der Blutgerinnung beteiligte Faktoren, insbesondere Protein C, Protein S und Fibrinogen, wenn überhaupt, nur in sehr geringem Ausmaß sorbiert werden.

Es ist ferner eine Aufgabe der Erfindung, ein Sorptionsmittel bereitzustellen, welches einfach und ökonomisch herstellbar ist.

Die Aufgaben werden durch ein Sorptionsmittel der eingangs genannten Art gelöst, welches erfindungsgemäß eine poröse Sorptionsmatrix mit einer neutralen, hydrophoben Oberfläche, einer mittleren Porengröße von 11 bis 20 nm und einer mittleren Partikelgröße von 75 bis 150 µm umfasst, zur Behandlung von Erkrankungen und Zuständen, die mit einer erhöhten Konzentration wenigstens eines Entzündungsmediators, insbesondere Zytokins, assoziiert sind.

Die Erfindung stellt ein Sorptionsmittel mit deutlich verbesserten Selektivitätseigenschaften bereit, mit welchem einerseits toxische Bestandteile wie Entzündungsmediatoren mit hoher Effizienz aus einer Körperflüssigkeit entfernt werden, und andererseits eine unerwünschte Adsorption von wichtigen körpereigenen Stoffen wie z.B. Gerinnungsfaktoren umgangen bzw. auf einem Minimum gehalten wird. Dank der Erfindung können Erkrankungen und Zustände, die mit einer erhöhten Konzentration von Entzündungsmediatoren im Zusammenhang stehen, insbesondere eine Sepsis einschließlich schwerer septischer Komplikationen sowie Sepsis-ähnliche Zustände, effizient behandelt werden. Besonders vorteilhaft wird das Sorptionsmittel in einem extrakorporalen Perfusionssystem, d.h. einem Blut- und/oder Plasmareinigungssystem, eingesetzt, wobei eine besonders effektive Entfernung/Abreicherung der Entzündungsmediatoren aus der Körperflüssigkeit und eine außerordentlich erfolgreiche Behandlung von Erkrankungen, allem voran einer Sepsis, ermöglicht wird. Gleichzeitig werden die oben erwähnten Probleme im Zusammenhang mit der Blutgerinnung vermieden. Die Erfindung bringt somit deutliche Vorteile und Verbesserungen hinsichtlich der Biokompatibilität und der Patientensicherheit mit sich.

Weitere Vorteile des erfindungsgemäßen Sorptionsmittels sind dessen einfache und ökonomische Herstellung im Vergleich zu den oben genannten Immunadsorbern, da keine spezifischen Beschichtungen mit technisch aufwändig herzustellenden Biomolekülen wie z.B. Antikörpern oder ähnlichen spezifischen Liganden notwendig sind.

Die Erfinder haben in ihren Versuchen (siehe Beispiele unten) den überraschenden Sachverhalt festgestellt, dass bei einer hocheffizienten Eliminierung von Entzündungsmediatoren, insbesondere dem potenten pro-inflammatorischen Schlüsselmediator TNF-α, die Bindung von Protein C durch das erfindungsgemäße Sorptionsmittel deutlich geringer ist im Vergleich zu Polymeren größerer mittlerer Porengröße. Bei einer Porengröße von weniger als 11 nm sinkt die Adsorptionseffizienz für die zu entfernenden toxischen Bestandteile, d.h. Entzündungsmediatoren wie Zytokine, insbesondere TNF-α. Bei einer Porengröße von mehr als 20 nm wird die innere Oberfläche kleiner und die Adsorptionskapazität für die zu entfernenden toxischen Bestandteile sinkt, während Protein C zunehmend adsorbiert wird. Erfindungsgemäß liegt die mittlere Partikelgröße bei 75 bis 150 µm, da sich überraschenderweise herausstellte, dass sich dieser Partikelgrößenbereich als vorteilhaft hinsichtlich der Adsorption von Protein C und Fibrinogen auswirkt. Bei einer Partikelgröße von 75 bis 150 µm scheint die äußere Oberfläche des Sorptionsmittels noch klein genug zu sein, um Protein C, wenn überhaupt, nur in einem unwesentlich geringen Ausmaß zu sorbieren; allerdings ist sie offenbar immer noch groß genug, um die Diffusionswege für die zu adsorbierenden Substanzen klein zu halten.

Protein C, ein Vitamin K-abhängiges Protein im Blutplasma, ist ein wichtiger Regulator des Blutgerinnungsablaufs und hat eine antikoagulatorische Wirkung. Dank des erfindungsgemäßen Sorptionsmittels können die durch eine unerwünschte Protein C-Adsorption hervorgerufenen Gerinnungskomplikationen (Venenthrombosen, Lungenembolien) vermieden werden. Es kann davon ausgegangen werden, dass auch die Adsorption von Protein S deutlich niedriger ist, da Protein S (62 000 Da) ein ähnliches relatives Molekulargewicht wie das Protein C (69 000 Da) aufweist. Dieselben Überlegungen gelten für Gerinnungsfaktoren ähnlichen Molekulargewichts wie z.B. Faktor VII, Faktor IX und Faktor X. Das erfindungsgemäße Sorptionsmittel weist daher eine deutlich verbesserte Biokompatibilität im Vergleich zu den bekannten Sorptionsmitteln auf. Beispielsweise ist zur Erhöhung der Biokompatibilität keine zusätzliche Beschichtung des Sorptionsmittels (z.B. mit humanem Serumalbumin (HSA)) notwendig. Da sich mit HSA beschichtete Sorptionsmittel nicht ohne weiteres sterilisieren lassen, ist als weiterer Vorteil die vergleichsweise einfache Sterilisierbarkeit zu nennen.

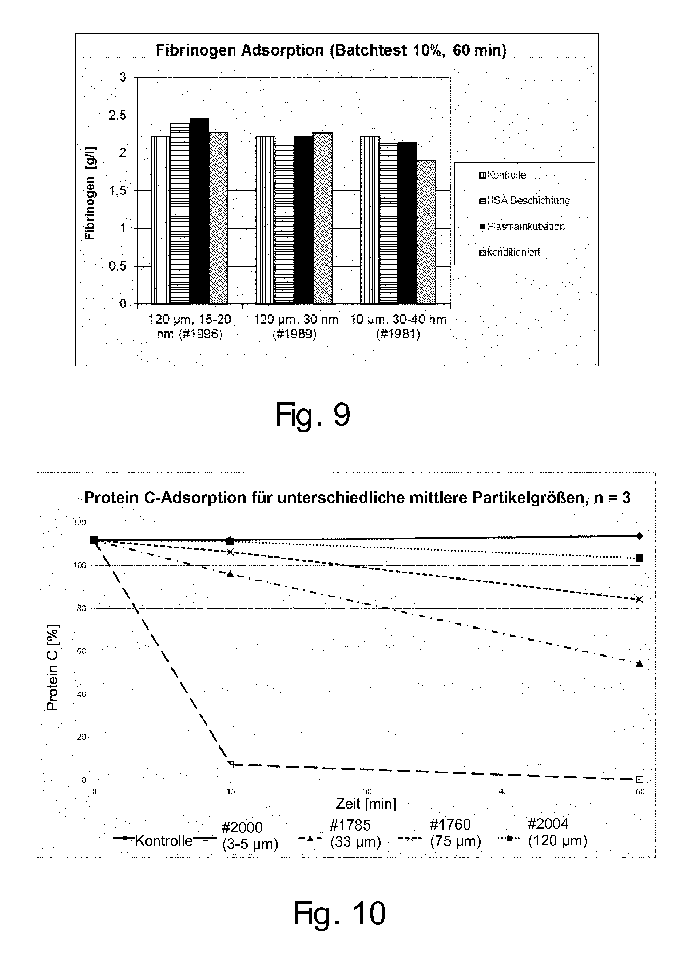

Die beobachtete geringe Adsorption wichtiger Gerinnungsfaktoren durch das erfindungsgemäße Sorptionsmittel auch bei längerer Inkubationsdauer ist überraschend und war auch nicht vorhersehbar. Die Erfinder konnten in ihren Versuchen den überraschenden Sachverhalt feststellen, dass die Adsorption von Protein C durch das erfindungsgemäße Sorptionsmittel signifikant gesenkt wird im Vergleich zu den bekannten Sorptionsmitteln. Die mittlere Partikelgröße beträgt erfindungsgemäß zumindest 75 µm. Bei einer mittleren Partikelgröße von 75 µm wurde die Protein C-Konzentration im Plasma auch nach längerer Inkubationsdauer (60 min) nur um ca. 25 % in Bezug auf die Ausgangskonzentration im Plasma gesenkt (vergleiche Beispiel 3/Fig. 10). Die mittlere Partikelgröße muss jedoch zumindest 75 µm betragen, da bei einer mittleren Partikelgröße von 75 µm gerade noch physiologisch relevante Mengen an Protein C auch nach längerer Inkubationsdauer in der Körperflüssigkeit verbleiben. Die Erfinder konnten feststellen, dass die Protein C-Adsorption mit sinkender mittlerer Partikelgröße signifikant zunimmt und die Protein C-Konzentration im Plasma physiologisch kritische Werte annimmt. Bei sehr klein gewählten mittleren Partikelgrößen wird Protein C fast gänzlich adsorbiert.

Die Erfinder konnten ferner feststellen, dass die Protein C-Adsorption mit ansteigender mittlerer Partikelgröße deutlich abnimmt (vergleiche Beispiel 3). Bei einer bevorzugten Ausführungsform weist die Sorptionsmatrix daher eine mittlere Partikelgröße von 100 bis 150 µm auf, da dadurch nochmals eine deutliche Verbesserung hinsichtlich der Protein C-Adsorption erreichbar ist. Mehr bevorzugt weist die Sorptionsmatrix eine mittlere Partikelgröße von 110 bis 130 µm, idealerweise eine mittlere Partikelgröße von ca. 120 µm, auf, da bei diesen Partikelgrößen einerseits toxische Substanzen wie Zytokine mit hoher Effizienz und andererseits wichtige Gerinnungsfaktoren wie Protein C und Fibrinogen kaum mehr adsorbiert werden, auch wenn die Körperflüssigkeit mit dem Sorptionsmittel über längere Zeit in Kontakt gebracht wird.

Die Adsorption wichtiger Gerinnungsfaktoren kann durch die gewählte mittlere Partikelgröße, die erfindungsgemäß in einem Bereich von 75 bis 150 µm liegt, beeinflusst werden. In einem Aspekt ist die Partikelgröße so gewählt, dass das Sorptionsmittel die Konzentration von Protein C um nicht mehr als 25 % in Bezug auf die Ausgangskonzentration des Protein C im Blut bzw. Blutplasma senkt. Durch Wahl einer größeren Partikelgröße kann dieser Prozentsatz verringert werden. Die Wahl der gewünschten Partikelgröße ist für einen Fachmann experimentell ohne unzumutbaren Aufwand ermittelbar. Die mittlere Partikelgröße ist vorzugsweise so gewählt, dass das erfindungsgemäße Sorptionsmittel die Konzentration des Protein C in einem Ausmaß von nicht mehr als 20 %, mehr bevorzugt von nicht mehr als 15 %, am meisten bevorzugt von nicht mehr als 10 % in Bezug auf die Ausgangskonzentration des Protein C im Blut bzw. Blutplasma senkt. Beispielsweise konnten die Erfinder in ihren Versuchen feststellen, dass bei einer mittleren Porengröße von 15 bis 20 nm und einer mittleren Partikelgröße von 120 µm die Protein C-Konzentration im Plasma auch nach längerer Inkubationsdauer (60 min) um nicht mehr als 10% gesenkt wurde.

Der Prozentsatz der Protein C-Adsorption steigt mit zunehmender Porengröße und abnehmender Partikelgröße an. Bei einer Porengröße von 30-40 nm ist eine sehr starke Protein C-Adsorption zu beobachten. Ein ähnliches Bild ergibt sich für Fibrinogen, wobei der Unterschied aber nicht so ausgeprägt ist wie bei Protein C.

Zusammenfassend kann festgestellt werden, dass die erfindungsgemäße spezifische Kombination aus einer mittleren Porengröße von 11 bis 20 nm und einer mittleren Partikelgröße von 75 bis 150 µm aufgrund der deutlich geringeren Adsorption wichtiger Gerinnungsfaktoren (z.B. Protein C und Fibrinogen) eindeutige Vorteile gegenüber den bislang bekannten Sorptionsmitteln aufweist. Das erfindungsgemäße Sorptionsmittel hat eine deutlich verbesserte Biokompatibilität und Selektivität gegenüber den bekannten Sorptionsmitteln, die ebenfalls auf Neutralharzen mit hydrophober Oberfläche beruhen.

Der Ausdruck "erhöhte Konzentration wenigstens eines Entzündungsmediators" bedeutet, dass zumindest ein Entzündungsmediator in einer Körperflüssigkeit (z.B. Blut oder Blutplasma) in einer Konzentration vorliegt, bei der nachteilige Wirkungen für den Patienten hervorgerufen werden. Als für die Erfindung repräsentative Entzündungsmediatoren sind Zytokine zu nennen. Beispielsweise handelt es sich bei dem Zytokin TNF-α um ein körpereigenes Signalmolekül, das eine wichtige regulative Rolle bei der Immunantwort und bei Entzündungsreaktionen innehat, welches jedoch bei einer erhöhten Konzentration in der Körperflüssigkeit (z.B. infolge eines Zytokinsturms, siehe oben) toxische Wirkung zeigt und mit der Pathologie zahlreicher Erkrankungen und Zustände, z.B. Sepsis und septischem Schock, in Verbindung steht. Als weitere repräsentative Beispiele sind die Zytokine IL-1β, IL-6, IL-8 und IL-10 zu nennen. Beispielsweise liegen bei der Sepsis die Werte für das TNF-α in der proinflammatorischen Phase zumindest über 100-200 ng/ml.

Der Begriff "poröse Sorptionsmatrix mit einer neutralen, hydrophoben Oberfläche" bezieht sich im Rahmen dieser Offenbarung auf einen porösen, wasserunlöslichen Feststoff, der äußere und innere Oberflächen aufweist. Die äußeren und inneren Oberflächen sind neutral und hydrophob. Unter dem Begriff "neutral" ist nicht-ionisch zu verstehen. Unter dem Begriff "innere Oberfläche" des Trägers wird die Gesamtheit der Oberflächen der Poren bezeichnet. Der Begriff "äußere Oberfläche" bezieht sich hingegen auf die Gesamtheit der Oberflächen des Trägers, die von außerhalb direkt zugänglich sind.

Unter dem Begriff "Sorptionsmittel" im Rahmen dieser Offenbarung ist ein Mittel zum Durchführen einer Sorption, vorzugsweise einer Adsorption, zu verstehen, d.h. Moleküle, die sich in einer biologischen Flüssigkeit befinden, werden durch die Oberflächenkräfte des Sorptionsmittels festgehalten. In der Beschreibung werden daher anstelle des Begriffs "Sorptionsmittel" auch die Begriffe "Adsorbtionsmittel" bzw. "Adsorbens" bzw. "Adsorber" verwendet.

Der im Rahmen der Erfindung verwendete Ausdruck "Körperflüssigkeit" bezieht sich auf alle Arten von Körperflüssigkeiten wie Blut, Blutplasma, Sekrete, Exkrete und dergleichen, insbesondere aber Blut und Blutplasma. Da es im Zuge einer extrakorporalen Blutreinigung auch notwendig ist, andere Flüssigkeiten wie beispielsweise Gerinnungshemmer enthaltende Lösungen (Heparin-Lösung, Citrat-Lösung) oder Substitutionslösungen (Elektrolyte, Flüssigkeiten zum Ausgleich des Flüssigkeitsverlust) in den extrakorporalen Blutkreislauf bzw. in einen Blutplasmakreislauf/Filtratkreislauf einzubringen, ist unter einer Körperflüssigkeit auch verdünntes Blut bzw. verdünntes Blutplasma zu verstehen. Die Erfindung ist hauptsächlich für den humanmedizinischen Bereich gedacht und bezieht sich daher primär auf menschliche Körperflüssigkeiten. Dies schließt jedoch nicht aus, dass die Erfindung auch für den veterinärmedizinischen Bereich geeignet ist.

Das Sorptionsmittel zeigt signifikante Vorteile bei der Behandlung von Erkrankungen und Zuständen, die mit einer erhöhten Konzentration wenigstens eines Entzündungsmediators, insbesondere eines Zytokins, assoziiert sind. Besonders vorteilhaft ist eine Behandlung mit dem Sorptionsmittel dann, wenn sich bereits klinische Symptome zeigen und mit einer rasch voranschreitenden Verschlechterung des Zustands des Patienten zu rechnen ist, beispielsweise bei einer Sepsis. In anderen Fällen, beispielsweise bei Patienten mit chronischem Leberversagen, kann die Behandlung auch dann sinnvoll sein, wenn eine erhöhte Konzentration wenigstens eines Entzündungsmediators nachweisbar ist, aber noch keine klinischen Symptome bemerkbar sind. Dadurch kann einer unkontrollierten Aktivierung des nukleärphagozytären Systems noch vor dem Auftreten klinischer Symptome entgegengewirkt werden.

Bei dem Entzündungsmediator handelt es sich vorzugsweise um einen potentiell schädlichen proinflammatorischen Entzündungsmediator, insbesondere um ein proinflammatorisches Zytokin. Repräsentative Beispiele für proinflammatorische Zytokine sind TNF-α, IL-1β, IL-6 und IL-8, wobei TNF-α als initialem proinflammatorischen Entzündungsmediator besondere Bedeutung zukommt. Das Sorptionsmittel ist daher besonders günstig zum Behandeln von Erkrankungen und Zuständen, die auf die toxischen Wirkungen von TNF-α zurückzuführen sind. Die unten angeführten Beispiele liefern den Beweis, dass TNF-α, IL-1β, IL-6, IL-8 sowie das anti-inflammatorische IL-10 durch das erfindungsgemäße Sorptionsmittel höchst effizient eliminiert werden und daher zur Behandlung von Erkrankungen und Zuständen, die mit einer erhöhten Konzentration wenigstens eines Entzündungsmediators, insbesondere eines Zytokins, assoziiert sind, außerordentlich gut geeignet sind.

Die Erkrankung bzw. der Zustand, der mit einer erhöhten Konzentration wenigstens eines Entzündungsmediators, insbesondere Zytokins, assoziiert ist, ist vorzugsweise ausgewählt ist aus der Gruppe bestehend aus Sepsis, insbesondere septischem Schock, Sepsis-ähnlichen Zuständen, Alkohol-Hepatitis und Autoimmunerkrankungen, insbesondere rheumatoider Arthritis, chronisch-entzündlicher Darmerkrankungen und Psoriasis.

Das erfindungsgemäße Sorptionsmittel ist bestens als Adsorbermaterial zur Behandlung einer Sepsis und deren schweren Verlaufsformen (schwere Sepsis, septischer Schock) sowie zur Behandlung von Sepsis-ähnlichen Zuständen geeignet. Wie eingangs erwähnt gehen septische Zustände mit stark erhöhten Zytokinkonzentrationen im Patientenblut einher. Eine effiziente Eliminierung bzw. Abreicherung des Schlüsselzytokins TNF-α ist dabei von besonderer Wichtigkeit. Der Begriff "Sepsis" wie hierin verwendet umfasst ferner alle Arten von septischen Komplikationen, z.B. Endotoxin-induzierte schwere Sepsis, septischer Schock, Multiorganversagen sowie schwere Verläufe des septischen Schocks wie Meningokokkensepsis, Waterhouse-Friderichsen-Syndrom etc. Der Ausdruck "Sepsis-ähnlicher Zustand" bezieht sich auf einen Zustand, der - biochemisch gesehen - einer Sepsis gleicht, d.h. mit einer unkontrollierten Aktivierung des mononukleär-phagozytären Systems einhergeht. Vorzugsweise ist der Sepsis-ähnliche Zustand ausgewählt aus der Gruppe bestehend aus akutem Leberversagen und akuter Dekompensation bei chronischem Leberversagen.

Bei akutem Leberversagen oder einer akuten Dekompensation bei chronischem Leberversagen ergeben sich Zustände, die - biochemisch gesehen - einer Sepsis sehr ähnlich sind. Beim Patienten mit intakter Leberfunktionen werden die aus dem Darm in die Blutbahn übertretenden Endotoxine vom Retikuloendothelialen System (RES) bzw. den Kupfferschen Sternzellen durch Endozytose eliminiert. Bei Patienten mit chronischem Leberversagen kann es zu einer akuten Dekompensation kommen. In diesem Fall können Endotoxine der normalen Darmflora die Darmbarriere überwinden und daher die Leber ungehindert passieren und im Körper die Freisetzung von Entzündungsmediatoren stimulieren. Folglich ist die Anwendung des erfindungsgemäßen Sorptionsmittels zur Behandlung einer akuten Dekompensation bei chronischem Leberversagen besonders vorteilhaft.

Die Behandlung von Patienten mit Sepsis bzw. Sepsis-ähnlichen Zuständen findet zweckmäßigerweise im Rahmen einer extrakorporalen Blut- und/oder Blutplasmareinigung statt.

Für die Praxis ist es besonders zweckmäßig, wenn der Träger ein neutrales, vorzugsweise synthetisches, Polymer ist. Dadurch kann eine gute Reproduzierbarkeit des Trägermaterials gewährleistet werden, insbesondere hinsichtlich der Porosität und der Partikelgröße. Die Porosität und die Partikelgröße lassen sich darüber hinaus sehr gut variieren. Bei dem Polymer kann es sich sowohl um ein Homopolymer als auch um ein Heteropolymer handeln.

Obwohl einem Fachmann auf dem Gebiet wohlbekannt ist, was unter dem Begriff "mittlere Porengröße" zu verstehen ist und wie die Porösität bzw. die mittlere Porengröße gezielt einstellt werden kann, soll dieser Begriff aus Gründen der Klarheit an dieser Stelle dennoch kurz definiert werden. Die mittlere Porengröße bezieht sich auf den mittleren Durchmesser der Poren. Bei einer gaußschen Größenverteilung der Porendurchmesser eines porösen Materials ist der mittlere Porendurchmesser jener, welchem dem Maximum der Verteilungskurve entspricht. Der mittlere Porendurchmesser kann beispielsweise mittels Stickstoffadsorption (wie beschrieben in Weber et al. 2008; Neutral styrene divinylbenzene copolymers for adsorption of toxins in liver failure. Biomacromolecules 9(4):1322-1328) oder mittels Quecksilberintrusion bestimmt werden. Eingestellt wird die Porengröße eines Polymers durch Variation der Konzentration der beteiligten Monomere bzw. Co-Monomere, des Lösungsmittels oder des Modulators. Je kleiner die Poren des Polymers gewählt sind, umso größer wird die innere Oberfläche des Polymers, die für eine Sorption, insbesondere Adsorption, zur Verfügung steht. Je größer die Poren, umso besser ist die Zugänglichkeit der Poren für größere Moleküle. Ein Herstellungsverfahren für ein synthetisches, hydrophobes Polymer definierter Porengröße, wie es für die Erfindung zur Anwendung kommen kann, ist in der oben genannten Publikation von Weber et al. beschrieben.

Für die praktische Durchführung haben sich vernetzte Polystyrol-Polymere und vernetzte Ethylvinylbenzol-Polymere als besonders günstig erwiesen. Bei der extrakorporalen Blutreinigung bestehen hohe Anforderungen an die Sterilität der Vorrichtungsbestandteile, welche mit den Körperflüssigkeiten des Patienten in Kontakt kommen. Dies gilt auch für Sorptionsmittel. Vernetzte Polystyrol-Polymere und vernetzte Ethylvinylbenzol-Polymere zeichnen sich durch eine hohe Stabilität gegenüber Hitze und Chemikalien aus und sind in der klinischen Praxis bereits etabliert. Bei einer bevorzugten Variante ist das vernetzte Polystyrol-Polymer ein Polystyrol-Divinylbenzol-Copolymer. Bei einer bevorzugten Variante ist das vernetzte Ethylvinylbenzol-Polymer ein Ethylvinylbenzol-Divinylbenzol-Copolymer.

Es ist natürlich auch möglich, anstelle des bevorzugten Polystyrol-Divinylbenzol-Copolymers bzw. Ethylvinylbenzol-Divinylbenzol-Copolymers andere Neutralharze hoher Hydrophobizität zu verwenden, welche einem einschlägigen Fachmann gut bekannt sind. Repräsentative Beispiele für andere neutrale, hydrophobe Polymere, die für die Erfindung geeignet sind, sind beispielsweise Polymere aus Styrol- und Ethylvinylbenzol-Monomeren, die mit Trivinylcyclohexan, Trivinylbenzol, Divinylnaphthalen, Divinylsulfon, Thrimethylolpropan-Triacrylat, Trimethylpropan-trimethacrylat vernetzt sind oder Harze auf Basis aliphatischer Ester sowie Mischungen davon.

In einem weiteren Aspekt der Erfindung ist die Oberfläche der Sorptionsmatrix mit einem endotoxinbindenden Lipopeptid, insbesondere Polymyxin, beschichtet. Dadurch können zusätzlich zu den Entzündungsmediatoren auch Endotoxine eliminiert werden. Die pathogene Wirkung der Endotoxine (Lipopolysaccharide) wurde bereits oben beschrieben. Es hat sich in Laborversuchen gezeigt, dass eine Beschichtung mit einem endotoxinbindenden Lipopeptid keine negativen Auswirkungen auf die Adsorption der Entzündungsmediatoren hat. Die endotoxinbindenden Lipopeptid-Moleküle können sowohl kovalent als auch über nichtkovalente, adsorptive Wechselwirkungen an der Oberfläche der Sorptionsmatrix immobilisiert sein, wobei eine Anbindung über nichtkovalente, adsorptive Wechselwirkungen bevorzugt wird. Bei dem endotoxinbindenden Lipopeptid handelt es sich vorzugsweise um Polymyxin. Polymyxine sind bekannte chemische Verbindungen, welche ursprünglich aus dem Bakterium *Bacillus polymyxa* stammen. Insbesondere sind Polymyxin B und Polymyxin E (Colistin) als für die vorliegende Erfindung geeignete Polymyxine hervorzuheben. Zum Beschichten der Oberfläche mit einem endotoxinbindenden Lipopeptid können bekannte Verfahren angewendet werden. Beispielsweise offenbaren die EP 0110 409A und die EP 0 129 786 A2 die kovalente Anbindung von Polymyxin-Molekülen auf Trägermaterialien. Ferner beschreibt die WO 2010/083545 die Herstellung eines Sorptionsmittels, an dessen Oberfläche Polymyxin über nichtkovalente Wechselwirkungen immobilisiert ist.

Besonders vorteilhaft ist die Verwendung des erfindungsgemäßen Sorptionsmittels als Füllmaterial für eine Sorptionseinrichtung. Ein weiterer Gegenstand der Erfindung ist daher eine Sorptionseinrichtung, welche wenigstens eine Art eines erfindungsgemäßen Sorptionsmittels enthält. Bei einer Ausführungsform enthält die Sorptionseinrichtung nur eine Art eines erfindungsgemäßen Sorptionsmittels. Bei einer weiteren Ausführungsform zwei oder mehr verschiedene Arten des Sorptionsmittels, z.B. Sorptionsmittel aus zwei oder mehr verschiedenen Arten nichtionischer Polymere, wobei die verschiedenen Sorptionsmittel entweder als Mischung oder in räumlich getrennten Kompartimenten in der Sorptionseinrichtung vorliegen können. Der Begriff "Sorptionseinrichtung" umfasst insbesondere eine Säule oder Kartusche, die mit den Sorptionsmittelpartikeln befüllt ist. Derartige Einrichtungen sind dem einschlägigen Fachmann bestens bekannt. Die zu reinigende Körperflüssigkeit, z.B. Blut oder Blutplasma, passiert dabei die Adsorptionssäule oder -kartusche, in welcher die toxischen Bestandteile gebunden und aus der Körperflüssigkeit entfernt werden. Für bestimmte Anwendungen ist es von Vorteil, wenn das partikelförmige Sorptionsmittel dispergierfähig ist.

Das erfindungsgemäße Sorptionsmittel kommt in erster Linie als Adsorbermaterial bei Aphereseverfahren, insbesondere für therapeutische Zwecke, zum Einsatz. Ein weiterer Gegenstand der Erfindung ist somit eine extrakorporale Perfusionsvorrichtung (Blut- und/oder Blutplasmareinigungsvorrichtung), die wenigstens ein erfindungsgemäßes Sorptionsmittel oder eine Sorptionseinrichtung wie oben genannt umfasst. Je nachdem, welche Vorrichtung bzw. welches Verfahren (Hämoperfusion, Plasmapherese/Plasmasorption) angewendet wird, kann die Sorptionseinrichtung blutseitig in einem extrakorporalen Blutkreislauf und/oder in einem Plasmakreislauf bzw. Filtratkreislauf auf der Filtratseite angeordnet sein. Die Körperflüssigkeit passiert die Sorptionseinrichtung, wobei die toxischen Bestandteile, insbesondere Entzündungsmediatoren wie Zytokine von der Sorptionsmatrix sorbiert werden. Das gereinigte Blut bzw. Blutplasma wird dem Patienten wieder zugeführt. Aphereseverfahren bzw. extrakorporale Blut- und Plasmareinigungsvorrichtungen, in welchem das hierin beschriebene Sorptionsmittel Anwendung finden kann, sind gemäß dem im Stand der Technik und dem einschlägigen Fachmann bekannten allgemeinen Prinzipien konzipiert bzw. konstruiert und beispielsweise in [Thomas Rimmele'and John A Kellum 2011: Clinical review: Blood purification for sepsis. Critical Care 15 (1): 205; Rafidah Atan, David Crosbie and Rinaldo Bellomo 2011: Techniques of Extracorporeal Cytokine Removal: A Systematic Review of the Literature. Blood Purification 33: 88-100] beschrieben.

Bei einer besonders vorteilhaften Ausführungsform einer extrakorporalen Perfusionsvorrichtung umfasst diese einen extrakorporalen Blutkreislauf und einen Filtratkreislauf, der mit dem extrakorporalen Blutkreislauf mittels eines Filters in Verbindung steht, wobei der Filter einen Siebkoeffizienten von 5 % für Substanzen mit einer relativen Molmasse von 340 kDa aufweist, und wobei die Sorptionseinrichtung im Filtratkreislauf angeordnet ist. Die Verwendung eines Filters mit einem Siebkoeffizienten von 5 % für Substanzen mit einer relativen Molmasse von 340 kDa (340 000 Da) bringt im Vergleich zur Behandlung ohne Filter (d.h. die Sorptionseinrichtung ist als Vollblutadsorber direkt blutseitig im extrakorporalen Blutkreislauf angeordnet) oder einem Plasmafilter, der nur die zellulären Blutbestandteile zurückhält, wesentliche Vorteile mit sich. Ein Filter mit einem Siebkoeffizienten von 5 % für Substanzen mit einer relativen Molmasse von 340 kDa lässt fraktioniertes Plasma durch, so dass hochmolekulare Blutbestandteile wie Fibrinogen, Immunglobuline, LDL, HDL etc. zurückgehalten werden, während kleinere Blutbestandteile wie Albumin oder Protein C die Filtermembran passieren. Es hat sich gezeigt, dass die Adsorptionsleistung für Zytokine, allen voran TNF-α, deutlich besser ist im Vergleich zu einem Plasmafilter, der nur die zellulären Blutbestandteile zurückhält, obwohl durch einen Filter mit einem Siebkoeffizienten von 5 % für Substanzen mit einer relativen Molmasse von 340 kDa weniger Zytokine in das fraktionierte Plasma gelangen. Es wird angenommen, dass der Nachteil des geringeren Siebkoeffizienten durch den Vorteil der dadurch bedingten Rückhaltung hochmolekularer Plasmabestandteile, insbesondere des Fibrinogens, überkompensiert wird und die Poren des in der Sorptionseinrichtung befindlichen Sorptionsmittels voll nutzbar bleiben. Ein für diese Ausführungsform geeigneter Filter ist unter dem Handelsnamen "Albuflow®" erhältlich (Hersteller: Fresenius Medical Care; Material: Polysulfon-Hohlfasern; Siebkoeffizient für Albumin von ≥ 0,6 und für Fibrinogen ≤ 0,1).

Bei einer ersten Variante ist der Filtratkreislauf offen und das fraktionierte Plasma wird nach Passage der Sorptionseinrichtung direkt dem extrakorporalen Blutkreislauf zugeführt. Das Grundprinzip dieser Variante kommt derzeit in der Immunadsorption zum Einsatz (beispielsweise Apheresevorrichtungen der Firma AsahiKasei, Japan).

Bei einer zweiten Variante ist der Filtratkreislauf geschlossen und das fraktionierte Plasma gelangt über die Membran des Albuflow® wieder in das im extrakorporalen Blutkreislauf strömende Blut. Das Grundprinzip der zweiten Variante ist durch das Blutreinigungssystem Prometheus® (Fresenius Medical Care GmbH, Deutschland) bekannt. [Falkenhagen D, Strobl W, Vogt G, Schrefl A, Linsberger I, Gerner FJ, Schoenhofen M.: Fractionated plasma separation and adsorption system: a novel system for blood purification to remove albumin bound substances. Artif Organs. 1999 Jan;23(1):81-6]

Die Erfindung betrifft ferner ein Verfahren zur Behandlung von Erkrankungen und Zuständen wie oben definiert, die mit einer erhöhten Konzentration wenigstens eines Entzündungsmediators, insbesondere Zytokine, assoziiert sind, durch Inkontaktbringen einer Körperflüssigkeit mit einem Sorptionsmittel wie hierin beschrieben, wobei das Sorptionsmittel eine poröse Sorptionsmatrix mit einer neutralen, hydrophoben Oberfläche, einer mittleren Porengröße von 11 bis 20 nm und einer mittleren Partikelgröße von 75 bis 150 µm umfasst. Der Begriff "Körperflüssigkeit" entspricht der oben angeführten Definition. Das Verfahren ist vorzugsweise als extrakorporales Blut- und/oder Blutplasmareinigungsverfahren wie in dieser Offenbarung beschrieben definiert.

Extrakorporale Perfusionssysteme wie oben beschrieben können aufgrund der verbesserten Adsorptionsleistung für Zytokine auch dann zweckmäßig sein, wenn ein Filter mit einem Siebkoeffizienten von 5 % für Substanzen mit einer relativen Molmasse von 340 kDa (z.B. ein Albuflow® Filter) mit einem weniger bevorzugten Sorptionsmittel kombiniert wird, beispielsweise mit einem Sorptionsmittel, welches eine mittlere Porengröße und/oder eine mittlere Partikelgröße aufweist, welche außerhalb des bevorzugten erfindungsgemäßen Bereichs liegen. Beispielsweise liegt die mittlere Porengröße in einem Bereich von 10-100 nm, mehr bevorzugt in einem Bereich von 10 bis 40 nm, noch mehr bevorzugt in einem Bereich von 10 bis 25 nm und am meisten bevorzugt in einem Bereich von 11 bis 20 nm. Beispielsweise liegt die mittlere Partikelgröße in einem Bereich von 3 bis 200 µm, vorzugsweise in einem Bereich von 75 bis 150 µm, mehr bevorzugt in einem Bereich von 100 bis 150 µm, am meisten bevorzugt in einem Bereich von 110 bis 130 µm und idealerweise bei 120 µm. Dies betrifft daher eine extrakorporale Perfusionsvorrichtung umfassend einen extrakorporalen Blutkreislauf und einen Filtratkreislauf, der mit dem extrakorporalen Blutkreislauf mittels eines Filters in Verbindung steht, wobei der Filter einen Siebkoeffizienten von 5 % für Substanzen mit einer relativen Molmasse von 340 kDa aufweist, und wobei im Filtratkreislauf eine Sorptionseinrichtung angeordnet ist, wobei in der Sorptionseinrichtung ein Sorptionsmittel umfassend eine poröse Sorptionsmatrix mit einer neutralen, hydrophoben Oberfläche angeordnet ist. Der Ausdruck "neutrale, hydrophobe Oberfläche" entspricht den oben genannten Definitionen; vorzugsweise ist die Sorptionsmatrix ein vernetztes Polystyrol-Polymer, vorzugsweise ein Polystyrol-Divinylbenzol-Copolymer oder ein Ethylvinylbenzol-Divinylbenzol Copolymer. Alternativ dazu kann das Sorptionsmittel auch als Suspension im fraktionierten Plasma vorliegen. Beispielsweise kommt eine Suspension des Sorptionsmittels in einem Microspheres-based Detoxificaton System (MDS), wie es in der EP 0776223 B1 beschrieben wurde, zur Anwendung. In einem MDS zirkulieren die Mikropartikel als Suspension in einem Filtratkreislauf auf der Filtratseite eines Membranfilters. Ein für diese Anordnung geeigneter Filter, der den oben genannten Kriterien entspricht, ist unter dem Handelsnamen "Albuflow®" erhältlich (Hersteller: Fresenius Medical Care; Material: Polysulfon-Hohlfasern; Siebkoeffizient für Albumin von ≥ 0,6 und für Fibrinogen ≤ 0,1).

Die vorliegende Erfindung wird im Folgenden anhand von nicht einschränkenden Beispielen näher erläutert.

### 1. BEISPIEL 1: Austesten von Ethylvinylbenzol-Divinylbenzol-Copolymeren verschiedener Porengröße hinsichtlich ihrer Adsorptionseigenschaften

Ethylvinylbenzol-Divinylbenzol-Copolymere unterschiedlicher Porengröße wurden hinsichtlich ihrer Adsorptionseigenschaften für Zytokine (TNF-α, IL-1β, IL-6, IL-8 und IL-10), untersucht und miteinander verglichen.

### 1.1. Bereitstellen neutraler, hydrophober Polymere

Zunächst wurden Ethylvinylbenzol-Divinylbenzol-Copolymere (im Folgenden auch als "Adsorber" bezeichnet) unterschiedlicher mittlerer Porengröße, bezogen von Rohm & Haas, bereitgestellt. Die Adsorber sind in der Tabelle 1 aufgelistet.

**Tabelle 1: Ethylvinylbenzol-Divinylbenzol-Copolymere**

| Bezeichnung des Ethylvinylbenzol-Divinylbenzol-Copolymers | Mittlere Porengröße [nm] |
|---|---|
| #1822 | 15-20 |
| #1823 | 15-20 |
| #1824 | 30-40 |
| #1825 | 80-100 |
| #1826 | 80-100 |

Die mittlere Partikelgröße der Polymere beträgt 5 µm +/- 3-4 µm.

### 1.2. Vorbereitung des gespikten Plasmas

TNF-α, IL-1β, IL-6, IL-8 und IL-10 sind in jeweils 1 mL PBS-0.1 %HSA-Puffer rekonstituiert, zu je 20 µL aliquotiert und bei -80 °C gelagert.

Die Stocklösung wird 1:10 in Plasma (fresh frozen plasma, Plasmaspendezentrum Retz) verdünnt (1:10; 5 µL Stock + 45 µL Plasma).

Die Endkonzentration des Plasmaspikes (50 mL) für die verwendeten Zytokine ist in der untenstehenden Tabelle 2 dargestellt:

**Tabelle 2:**

| | S**tock-Konzentration [µg/mL]** | S**tockverdünnung 1:10** | **Stockverd. zu Plasma [49905 µL]** | **Endkonzentration im Plasma [pg/mL]** |
|---|---|---|---|---|
| TNF-α | 10 | 5 µL + 45 µl | 7.5 µL | 500 |
| IL-1β | 5 | 5 µL + 45 µl | 32.5 µL | 250 |
| IL-6 | 10 | 5 µL + 45 µl | 17.5 µL | 200 |
| IL-8 | 10 | 5 µL + 45 µl | 17.5 µL | 200 |
| IL-10 | 10 | 5 µL + 45 µl | 20 µL | 300 |

### 1.3. Zytokinadsorption - Batchtest

Die in Tabelle 1 aufgelisteten Adsorber wurden in einem Batchtest hinsichtlich der Adsorption der Zytokine TNF-α, IL-1β, IL-6, IL-8 und IL-10 untersucht und miteinander verglichen.

Jeder Adsorber sowie eine Kontrolle (Plasma ohne Adsorber) wurden im Doppelansatz getestet.

Jeder Ansatz enthält 10 % (v/v) Adsorber (0,5 ml Adsorber Feuchtvolumen und 4,5 ml Plasmaspike) in einem Teströhrchen. Als Kontrolle wurde ein Teströhrchen ohne Adsorber mitgeführt.

Die Probenahmen erfolgten zu folgenden Zeitpunkten: 0, 15 und 60 Minuten.

Zu diesen Zeitpunkten wurden jeweils 1 ml Adsorber-Plasmasuspension entnommen, zentrifugiert (Eppendorf-Zentrifuge: 14000 rpm, 3 min) und der Überstand aliquotiert (1x 200 µl, 1x 700 µl) und bei -18°C bis zur Analyse eingefroren. Für die Verdünnung der Standardkurve wurden 5 ml natives Plasma eingefroren.

### 1.4. Analysen und Ergebnisse

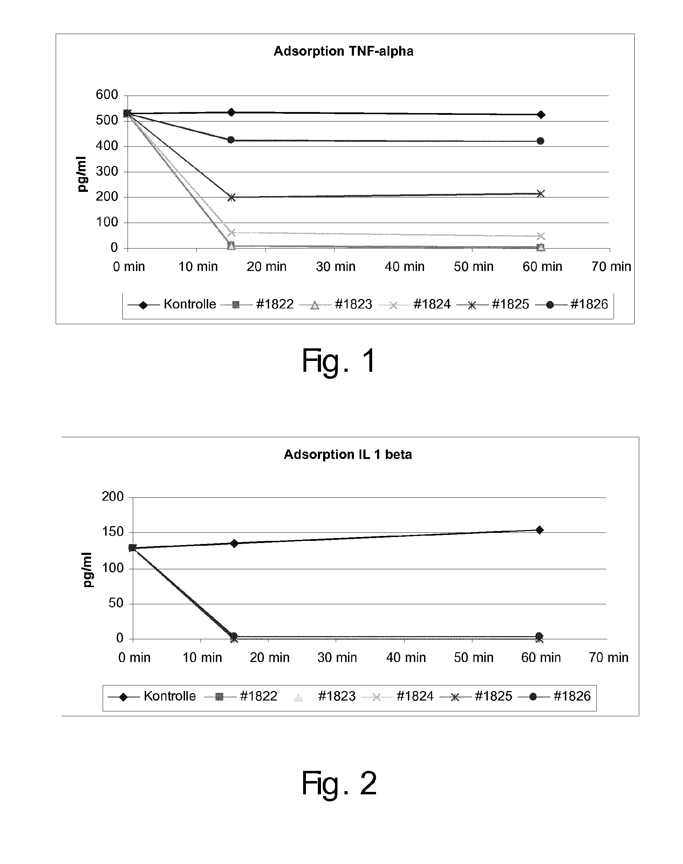

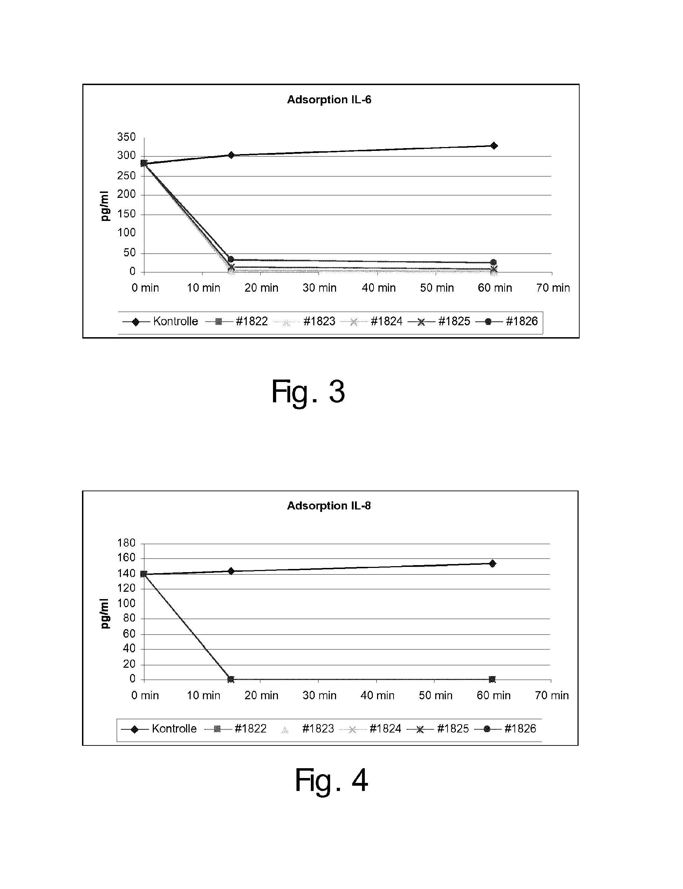

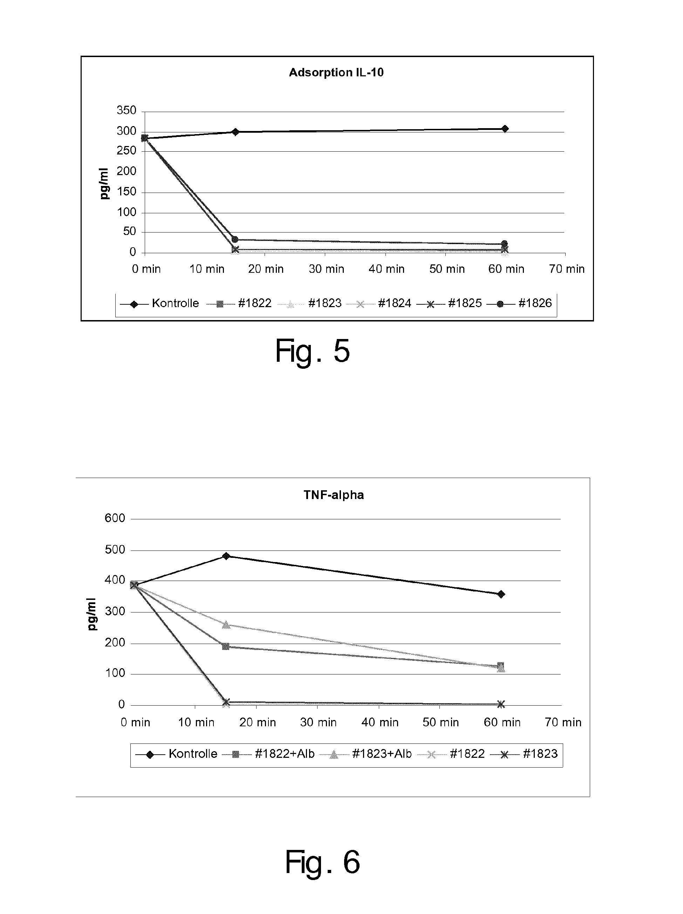

Die Bestimmung der Zytokinkonzentrationen erfolgte mit einem Luminex Bead Array System (Biorad). Die Adsorption der Zytokine TNF-α (Fig. 1), IL-1β (Fig. 2), IL-6 (Fig. 3), IL-8 (Fig. 4) und IL-10 (Fig. 5) durch die Adsorber im Zeitverlauf sind in den Fig. 1 bis 5 dargestellt. Aus der Fig. 1 ist eindeutig zu erkennen, dass TNF-α am effizientesten durch Mikropartikel mit einer mittleren Porengröße von 15-20 nm aus dem gespikten Plasma entfernt wird.

Es hat sich in weiteren Versuchen herausgestellt, dass bei kleinen mittleren Partikelgrößen und einer Porengröße von 15-20 nm Protein C vermehrt adsorbiert wird (vergleiche Bsp. 3). Durch eine Beschichtung der Adsorber #1822 und #1823 mit Humanem Serum Albumin (HSA) gemäß Protokoll unter Punkt 2.2. (siehe unten) wurden HSA-beschichtete Adsorber erhalten (als #1822+Alb und #1823+Alb bezeichnet). Durch die HSA-Beschichtung konnte die Protein C-Adsorption gesenkt werden. Allerdings wirkt sich die Beschichtung mit HSA nachteilig auf die Adsorption des TNF- α aus (siehe Fig. 6).

### 2. BEISPIEL 2: Austesten von Ethylvinylbenzol-Divinylbenzol-Copolymeren verschiedener Porengröße und verschiedener Partikelgröße hinsichtlich ihrer Adsorptionseigenschaften

Ethylvinylbenzol-Divinylbenzol-Copolymere mit unterschiedlicher mittlerer Porengröße und unterschiedlicher mittlerer Partikelgröße wurden unterschiedlich vorbehandelt und im 3-fach Ansatz im Batchtest hinsichtlich ihrer Adsorptionseigenschaften für TNF-α, Protein C und Fibrinogen untersucht und miteinander verglichen. Es soll gezeigt werden, ob Plasmaproteine die Adsorptionsleistung von Zytokinen beeinflussen. Das Hauptaugenmerk liegt bei der Anlagerung von Albumin an die Adsorberoberfläche. Da in der klinischen Anwendung in einem extrakorporalen Perfusionssystem der Adsorber mit Plasma ständig umflossen wird, kann die Anlagerung von Plasmaproteinen die Zytokinadsorption beeinflussen. Dies passiert bei kleinporigen Adsorbern nicht, da durch die Siebwirkung die größeren Proteine nicht in Kontakt mit der inneren Oberfläche des Adsorbers kommen. Dies wird im Versuch durch Inkubation in HSA-Lösung (HSA = Humanes Serumalbumin) als auch durch Inkubation mit Plasma gezeigt. Bei den Adsorbern mit größeren Poren (30 - 40 nm) lässt die Zytokinadsorption durch das Vorinkubieren in Plasma bzw. HSA-Lösung deutlich nach.

### 2.1. Bereitstellen neutraler, hydrophober Polymere

Die in diesem Beispiel verwendeten Ethylvinylbenzol-Divinylbenzol-Copolymere (im Folgenden als "Adsorber" bezeichnet) mit unterschiedlicher mittlerer Porengröße und unterschiedlicher mittlerer Partikelgröße sind in der Tabelle 3 aufgelistet.

**Tabelle 3: Ethylvinylbenzol-Divinylbenzol-Copolymere**

| Bezeichnung des Ethylvinylbenzol-Divinylbenzol-Copolymers | Mittlere Porengröße [nm] | Mittlere Partikelgröße [µm] |
|---|---|---|
| #1996 | 15-20 | 120 |
| #1989 | 30 | 120 |
| #1981 | 30 - 40 | 10 |

### 2.2. Adsorbervorbereitung und Batchtest

Die in Tabelle 3 genannten Adsorber #1996, #1989 und #1981 wurden im 3-fach Ansatz im Batchtest hinsichtlich ihrer Adsorptionseigenschaften für TNF-α, Protein C und Fibrinogen untersucht und miteinander verglichen. Die Adsorber wurden unterschiedlich vorbehandelt und in 3 verschiedenen 10%igen Ansätzen eingesetzt.

*Ansatz* 1: Adsorber, der mit HSA beschichtet wurde. Für die HSA-Beschichtung wird eine entsprechende Menge 10 %ige HSA-Lösung hergestellt. Der konditionierte Adsorber wird abzentrifugiert (4000 g, 10 min), der Überstand verworfen und pro ml Adsorbersediment 2,5 ml HSA-Lösung für die Beschichtung zupipettiert. Die Adsorbersuspension wird mindestens 12 h bei Raumtemperatur mit der HSA-Lösung inkubiert (Enviro Genie Schüttler, Frequenz 25:50). Nach der Inkubationszeit wird der HSA-beschichtete Adsorber 1-mal mit 5 fachem Volumen 0,9 %iger NaCl-Lösung gewaschen. Der Adsorber wird schlussendlich als 50 %ige Suspension in 0,9 % NaCl im Kühlschrank gelagert.

*Ansatz* 2: Adsorber, der in nativem Citratplasma vorinkubiert wurde. 120 µl Adsorber in 300 µl Citratplasma (entspricht Verhältnis der HSA-Beschichtung mit 1 ml Adsorber und 2,5 ml 10 %iger HSA-Lösung) werden für 10 min direkt vor dem Batchtest inkubiert.

*Ansatz* 3: Adsorber, der vorher nur konditioniert wurde. Konditionierung von Adsorbern: Trockene Adsorber sollten vor der Verwendung konditioniert werden, um eine gute Benetzung mit wässrigen Lösungen bzw. mit Plasma zu ermöglichen. Trockene, hydrophobe Adsorber werden folgendermaßen vorbehandelt: Die benötigte Menge trockener Adsorber wird in ein 50 ml Greiner-Röhrchen gegeben und mit dem 5-fachem Volumen unvergälltem Ethanol gewaschen (suspendiert und 5 min bei 4000 rpm zentrifugiert). Der Überstand wird abgehoben und verworfen und mit frischem, unvergälltem Ethanol wieder suspendiert und für 1 h inkubiert (Enviro Genie, Frequenz 25:50). Nach der Inkubation wird die Adsorbersuspension abzentrifugiert (5 min bei 4000 rpm zentrifugiert) und der Überstand verworfen. Der Adsorber wird nun mit dem 5-fachem Volumen destilliertem Wasser gewaschen (suspendiert und 5 min bei 4000 rpm zentrifugiert). Der Überstand wird abgehoben und verworfen und mit frischem destilliertem Wasser wieder suspendiert und für 1 h inkubiert (Enviro Genie, Frequenz 25:50). Nach der Inkubation wird die Adsorbersuspension abzentrifugiert (5 min bei 4000 rpm zentrifugiert) und der Überstand verworfen. Der Adsorber wird nun mit dem 5-fachem Volumen physiologischer Kochsalzlösung gewaschen (suspendiert und 5 min bei 4000 rpm zentrifugiert). Der Überstand wird abgehoben und verworfen und mit frischer physiologischer Kochsalzlösung wieder suspendiert und für 1 h inkubiert (Enviro Genie, Frequenz 25:50). Nach der Inkubation wird die Adsorbersuspension abzentrifugiert (5 min bei 4000 rpm zentrifugiert) und der Überstand verworfen. Zuletzt wird mit physiologischer Kochsalzlösung eine 50 %ige Adsorbersuspension hergestellt und bis zur Verwendung im Kühlschrank aufbewahrt.

Für den Zytokinspike wurden 50 ml Citratplasma mit erwarteten 500 pg/ml TNF-α verwendet. TNF-α wurde in 1 ml PBS-0.1 % HSA-Puffer rekonstituiert, zu 20 µl aliquotiert und bei - 80 °C gelagert (Lot: AA3309093) (siehe Tabelle 4).

**Tabelle 4:**

| | **Stock-Konzentration [µg/mL]** | **Stockverdünnung 1:10** | **Stockverd. zu Plasma [40000 µL]** | **Laut Vorversuch erwartete Endkonzentration im Plasma [pg/mL]** |
|---|---|---|---|---|
| TNF-α | 10 | 5 µL + 45 µl | 6 µL | 500 |

Die vorbehandelten Adsorber wurden mit gespiktem Plasma überschichtet, wobei 120 µl Adsorber mit 1080 µl gespiktem Plasma versetzt wurden. Es wurden 3 verschiedene 10 %ige Ansätze hergestellt. Als Kontrolle wurde ein Teströhrchen mit gespiktem Citratplasma (Citratplasma mit TNF-α) ohne Adsorber mitgeführt.

Probennahme: Nach 60 min Inkubation wurde der Adsorber abzentrifugiert, der Überstand (Plasma) in je 2 Röhrchen (Eppendorf) aliquotiert und bis zur Analytik eingefroren.

### 2.3. Analysen und Ergebnisse

Die Konzentration von TNF-α wurde mittels kommerziell erhältlichen TNF-ELISA (Human TNF-α Immunoassay, DTA00C, R&D Systems®) und eines Microplate Reader (Anthos hat-III) bestimmt.

Protein C und Fibrinogen wurden am Sysmex (Siemens, CA560) mit den dazugehörigen Reagenzien (Siemens, OUVV17) analysiert.

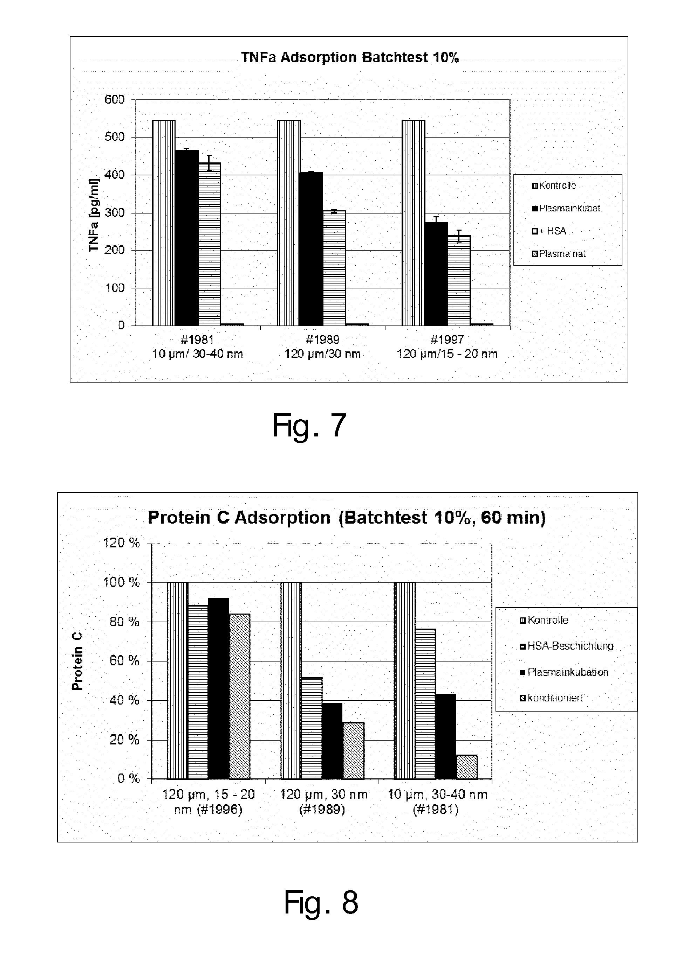

Die Adsorption von TNF-α (Fig. 7), Protein C (Fig. 8) und Fibrinogen (Fig. 9) durch die Adsorber nach 60 min Inkubation ist in den Fig. 7 bis 9 dargestellt. Aus dem Vergleich der erfindungsgemäßen Adsorberpartikel #1996 (mittlere Porengröße von 15 bis 20 nm, mittlerer Partikeldurchmesser von 120 µm) mit den Adsorberpartikeln #1989 (mittlere Porengröße von 30 nm, mittlerer Partikeldurchmesser von 120 µm) und #1981 (mittlere Porengröße von 30-40 nm, mittlerer Partikeldurchmesser von 10 µm) geht eindeutig hervor, dass Protein C vom erfindungsgemäßen Adsorber #1996 mit einer Porengröße von 15 bis 20 nm nicht signifikant adsorbiert wird. Die Adsorptionsrate für Protein C bei den Adsorbern #1989 und #1981 mit einer Porengröße von 30 bzw. 30-40 nm ist hingegen besonders ausgeprägt. Die Adsorber #1989 und #1981 weisen eine marginal höhere Adsorptionsrate für TNF-α als der Adsorber #1996 auf, die auf eine bessere Zugänglichkeit zu den inneren Poren erklärt werden kann. Der große Vorteil der erfindungsgemäßen Adsorberpartikel #1996 liegt somit darin, dass Protein C nicht wesentlich adsorbiert wird.

### 3. BEISPIEL 3: Austesten von Ethylvinylbenzol-Divinylbenzol-Copolymeren gleicher Porengröße und verschiedener Partikelgröße hinsichtlich ihrer Adsorptionseigenschaften

Ethylvinylbenzol-Divinylbenzol-Copolymere (Adsorber) mit gleichen mittleren Porengrößen (15-20 nm), aber mit unterschiedlichen mittleren Partikelgrößen von 3-5 µm, 35 µm, 75 µm bzw. 120 µm werden hinsichtlich der Adsorptionseigenschaft für Protein C verglichen.

### 3.1. Bereitstellen neutraler, hydrophober Polymere

Die in diesem Beispiel verwendeten Ethylvinylbenzol-Divinylbenzol-Copolymere mit gleicher mittlerer Porengröße und unterschiedlicher mittlerer Partikelgröße sind in der Tabelle 5 aufgelistet.

**Tabelle 5: Ethylvinylbenzol-Divinylbenzol-Copolymere**

| Bezeichnung des Ethylvinylbenzol-Divinylbenzol-Copolymers | Mittlere Porengröße [nm] | Mittlere Partikelgröße [µm] |
|---|---|---|
| #2000 | 15-20 | 3-5 |
| #1785 | 15-20 | 35 |
| #1760 | 15-20 | 75 |
| #2004 | 15-20 | 120 |

### 3.2. Adsorbervorbereitung und Batchtest

Die in Tabelle 5 genannten Adsorber #2000, #1785, #1760 und und #2004 wurden im Batchtest hinsichtlich ihrer Adsorptionseigenschaften für Protein C untersucht und miteinander verglichen.

Die Adsorber wurden konditioniert (siehe Beispiel 2/Punkt 2.2.)und direkt vor dem Batchtest für 15 min in Plasma inkubiert, abzentrifugiert und anschließend im Batchtest eingesetzt.

Für den Batchansatz (Dreifachansatz, n=3) wurden je 150µl Adsorber (feucht) mit 1350 µl Citratplasma in 15er Greinerröhrchen überschichtet. Die Röhrchen wurden im Enviro-Genie mit 25/50 rpm bei 37°C für 60 min geschüttelt. Als Kontrolle (120 µl NaCl + 1350 µl Citratplasma) wurde ein Röhrchen ohne Adsorber mitgeführt.

Nach 15 min und nach 60 min wurden für die Protein C-Analyse Proben zu je 500 µl gezogen. Protein C wurde am Sysmex (Siemens, CA560) mit den dazugehörigen Reagenzien (Siemens, OUVV17) analysiert.

### 3.3. Analysen und Ergebnisse

Fig. 10 zeigt die Protein C-Konzentration (Angabe in [%] in Bezug auf die physiologische Protein-C Konzentration in humanem Plasma) im Zeitverlauf für die einzelnen Adsorber. Anhand der Kurven zeigt sich sehr deutlich die Abhängigkeit der Protein C-Adsorption von der mittleren Partikelgröße. Eine ausgeprägte Protein C-Adsorption wurde bei den Adsorbern #2000 und #1785 festgestellt. Beim Adsorber #2000 war Protein C bereits nach 15 Minuten fast gänzlich aus dem Plasma entfernt. Durch die Adsorber #1760 und #2004 hingegen wurde Protein C in einem wesentlich geringeren Ausmaß aus dem Plasma adsorbiert. Die für den Adsorber #1760 beobachtete Protein C-Abnahme von - 25% (nach 60 min Inkubation) befindet sich gerade noch in einem Bereich, in welchem physiologisch relevante Mengen an Protein C im Plasma verbleiben. Die geringste Protein C-Adsorption, welche nach 60 min Inkubation lediglich 8% in Bezug auf die Protein C-Ausgangskonzentration betrug, wurde für Adsorber #2004 festgestellt. Die Protein C-Adsorption (in % bezogen auf die Protein C-Ausgangskonzentration) durch die einzelnen Adsorber ist in der Tabelle 6 aufgelistet.

**Tabelle 6:**

| Adsorber: | Protein-C-Adsorption nach 15 min Inkubation | Protein C-Adsorption nach 60 min Inkubation |
|---|---|---|
| #2000 | 94 % | 99 % |
| #1785 | 14 % | 52% |
| #1760 | 5% | 25 % |
| #2004 | 1% | 8% |

### 4. BEISPIEL 4: Austesten eines erfindungsgemäßen Adsorbers in einem Minisystem-Setup

Die Effizienz eines Ethylvinylbenzol-Divinylbenzol-Copolymers (Adsorber) mit einer Partikelgröße von 120 µm und einer mittleren Porengröße von 15 bis 20 nm (Bezeichnung: #1997) wurde hinsichtlich der Adsorptionseigenschaften für Zytokine in einem Minisystem-Setup untersucht.

### 4.1. Systemaufbau

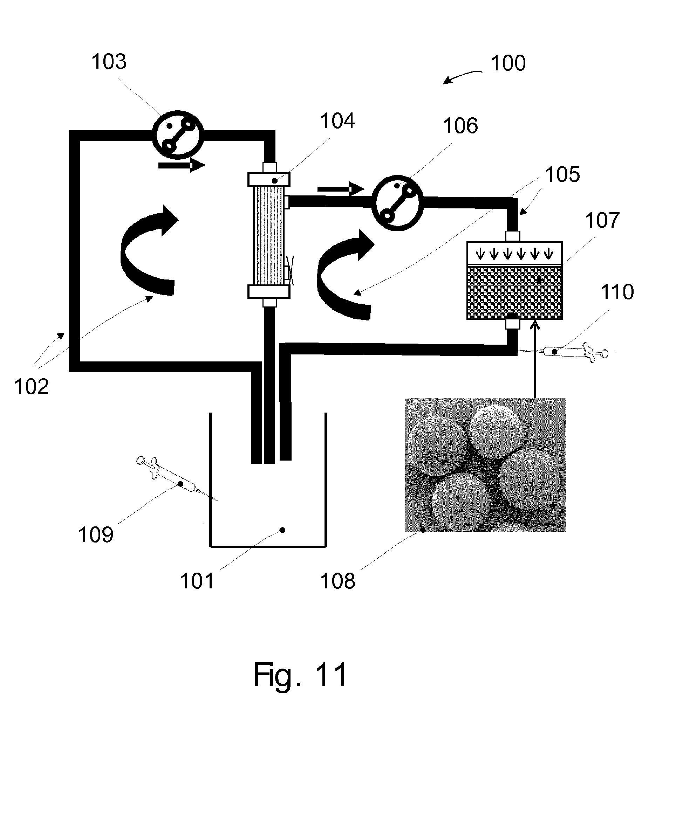

Der Systemaufbau 100 ist in der Fig. 11 schematisch dargestellt. Das in einem Gefäß 101 befindliche Blut (100 ml Blutprobe) wird in einem Blutkreislauf 102 mittels einer Blutpumpe 103 (Pumprate: 50 ml/min) durch einen Plasmafilter 104 und von dort wieder zurück in das Gefäß 101 geführt. Im Plasmafilter 104 wird ein Teil des Blutplasmas abfiltriert und einem Plasmakreislauf 105 zugeführt. Das im Plasmakreislauf 105 geführte Blutplasma wird mittels einer Plasmapumpe 106 (Pumprate: 10 ml/min) durch eine mit porösen Adsorberpartikeln befüllte Adsorptionseinrichtung 107 und schließlich zurück in das Gefäß 101 gepumpt. In der Adsorptionseinrichtung 107 befinden sich insgesamt 5 ml einer Suspension an Adsorberpartikeln, wobei die Adsorberpartikel eine mittlere Partikelgröße von 120 µm und eine mittlere Porengröße von 15 bis 20 nm aufweisen (als Adsorber #1997 bezeichnet). Die Adsorptionseinrichtung ist im Minisystem-Versuchsaufbau als Tropfkammer ausgeführt. Die Fig. 11 zeigt ferner zu Veranschaulichungszwecken unter dem Bezugszeichen 108 eine mikroskopische Aufnahme von 120 µm-Mikropartikeln.

An den in Fig. 11 gekennzeichneten Positionen 109 und 110 werden in bestimmten Zeitabständen die Proben zum Bestimmen der Zytokinkonzentrationen gezogen.

Als Kontrollsystemaufbau (nicht dargestellt) wird der gleiche Systemaufbau wie in Fig. 11 verwendet, wobei der Kontrollsystemaufbau jedoch keine Adsorberpartikel aufweist.

### 4.2. Adsorbervorbereitung und Blutprobe

### Adsorberkonditionierung:

Der Adsorber #1997 (Ethylvinylbenzol-Divinylbenzol-Copolymer, 120 µm, 15-20 nm) wird in eine Kartusche (Empty Rezorian™ Tube Kit mit PE-Fritten, Volumen 5 mL, 57613-U, Sigma-Aldrich) gefüllt. Die befüllte Adsorberkapsel wird mit 5 ml Ethanol absolut gespült und mit Ethanol gefüllt und an beiden Seiten verschlossen eine Stunde lang in das Ultraschallbecken gestellt. Danach wird die Kapsel mit 20 ml Aqua dest gespült, mit Aqua dest gefüllt wieder für eine Stunde im Ultraschallbecken inkubiert und das gleiche Prozedere mit NaCl-Lösung durchgeführt. Schlussendlich wird die Kapsel nochmals mit 20 ml NaCl-Lösung gespült und als feuchte in den Sekundärkreislauf (möglichst steril) eingespannt.

### Systemsterilisation:

Die beiden Kreisläufe werden am Tag vor dem Versuch vollständig aufgebaut und mit 70 % Ethanol absolut luftfrei gefüllt und über Nacht inkubiert. Am Versuchstag werden die beiden Kreisläufe mit je 1 L NaCl phys. gespült (single pass) und mindestens 15 min rezirkuliert. Vor Versuchsstart wird das gesamte System leer gepumpt um den Verdünnungseffekt so gering wie möglich zu halten.

### Zytokinspike:

TNF-α, IL-1β, IL-6, IL-8 und IL-10 sind in jeweils 1 mL PBS-0.1%HSA-Puffer rekonstituiert, zu 20 µL aliquotiert und bei -80°C gelagert.

Die Endkonzentrationen der Zytokine im Plasma nach Zugabe zum Vollblut sind in der Tabelle 7 angegeben.

**Tabelle 7:**

| | R&D Systems^{®} Lot Nr: | **Stock-Konzentration [µg/mL]** | **Stock. zu Vollblut [100 mL]** | **Laut Vorversuch erwartete End-konzentration im Plasma [pg/mL]** |
|---|---|---|---|---|
| TNF-α | AA3309093 | 10 | 1,5 µL | 500 |
| IL-1β | AD1410071 | 5 | 6,5 µL | 250 |
| IL-6 | OJZ0410052 | 10 | 3,5 µL | 200 |
| IL-8 | BEC0109041 | 10 | 3,5 µL | 200 |
| IL-10 | EYB016011 | 10 | 4 µL | 300 |

### Probennahme:

An den in Fig. 11 gekennzeichneten Positionen 109 und 110 werden zu den folgenden Zeitpunkten Proben genommen: 0, 10, 15, 30, 60, 120 und 240 Minuten.

Probenmengen: 1 ml Probe ziehen, abzentrifugieren und Plasmaüberstand aufteilen auf je 2 Eppendorfröhrchen: 30 µl für Zytokinbestimmung und 500 µl für die Bestimmung des Protein C. (mittels Sysmex durchgeführt).

### 4.3. Analysen und Ergebnisse

Die Bestimmung der Zytokinkonzentrationen erfolgte mit einem Luminex Bead Array System (Biorad).

Die Bestimmung der Protein C-Konzentration erfolgte am Sysmex (Siemens, CA560) mit den dazugehörigen Reagenzien (Siemens, OUVV17).

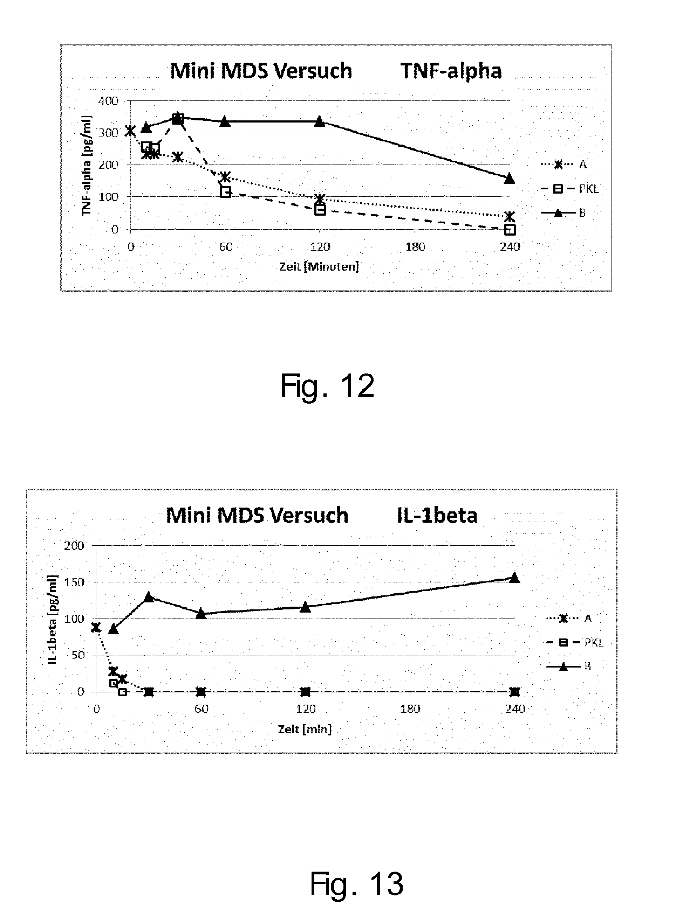

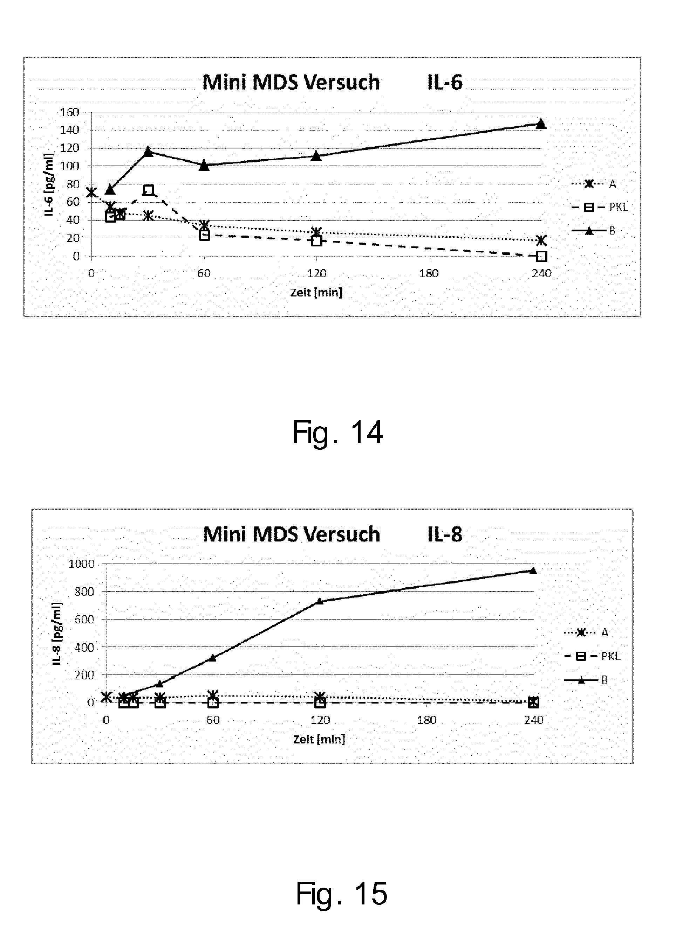

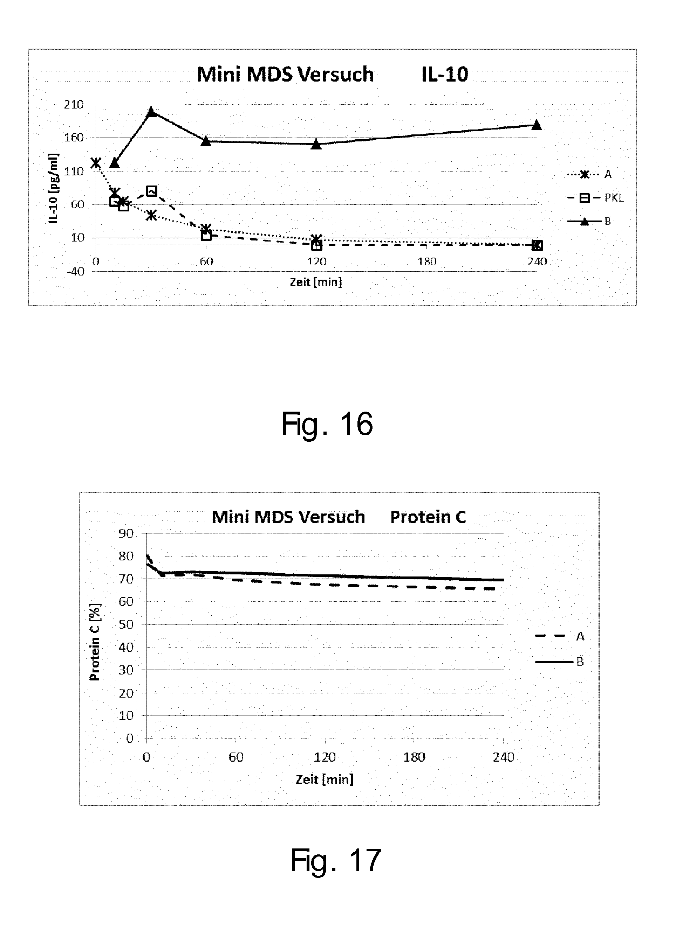

Die Adsorption der Zytokine TNF-α (Fig. 12), IL-1β (Fig. 13), IL-6 (Fig. 14), IL-8 (Fig. 15) und IL-10 (Fig. 16) und des Protein C (Fig. 17) im Zeitverlauf sind in den Fig. 12 bis 17 dargestellt, wobei die Balken zeigen:
A: Probennahme an der Position 109 (Blutprobe aus Gefäß 101) im Systemaufbau 100 wie in Fig. 11 dargestellt.
B: Probennahme im Kontrollsystem, welches keine Adsorberpartikel aufweist, an einer Position entsprechend der Position 109 im Systemaufbau 100 (Blutprobe aus Gefäß 101).
PKL: Probennahme an Position 110 im Systemaufbau 100 wie in Fig. 11 dargestellt (Plasmaprobe aus Plasmakreislauf 105 stromab der Adsorptionseinrichtung 107).

### 5. BEISPIEL 5: Extrakorporale Perfusionsvorrichtung mit offenem Filtratkreislauf

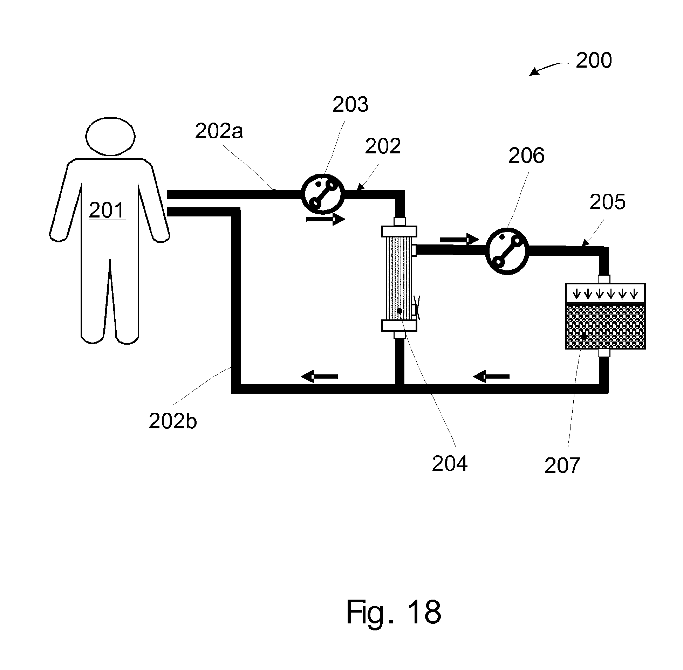

Die Fig. 18 zeigt eine schematische Darstellung einer extrakorporalen Perfusionsvorrichtung 200 (extrakorporale Blutreinigungsvorrichtung 200). Die Perfusionsvorrichtung 200 weist einen extrakorporalen Blutkreislauf 202 mit einem arteriellen Zulauf 202a (arterieller Schenkel) von einem Patienten 201 zu einem Filter 204 und einen venösen Ablauf 202b (venöser Schenkel) vom Filter 204 zum Patienten 201 auf. Das Patientenblut wird mittels einer Blutpumpe 203 (Pumprate Q_{Blut} = 200 ml/min) im Blutkreislauf 202 geführt. Der Filter 204 hat einen Siebkoeffizienten von 5 % für Substanzen mit einer relativen Molmasse von 340 kDa und ist beispielsweise ein Filter des Typs Albuflow® (Hersteller: Fresenius Medical Care; Material: Polysulfon-Hohlfasern; Siebkoeffizient für Albumin von ≥ 0,6 und für Fibrinogen ≤ 0,1). Durch den Filter 204 wird ein Teil des Blutplasmas (= fraktioniertes Plasma) abfiltriert und einem Filtratkreislauf 205 zugeführt. Ein Filter mit einem Siebkoeffizienten von 5 % für Substanzen mit einer relativen Molmasse von 340 kDa lässt fraktioniertes Plasma durch, so dass hochmolekulare Blutplasmabestandteile wie Fibrinogen, Immunglobuline, LDL, HDL etc. zurückgehalten werden, während kleinere Blutbestandteile wie Albumin oder Protein C die Filtermembran passieren. Der Filtratkreislauf 205 ist als offener Kreislauf realisiert, wobei das im Filtratkreislauf 205 geführte fraktionierte Blutplasma mittels einer Filtratpumpe 206 (Pumprate Q_{frakt. Plasma} = 30-60 ml/min) durch eine mit erfindungsgemäßen Adsorberpartikeln befüllte Adsorptionseinrichtung 207 und schließlich stromab der Adsorptionseinrichtung 207 in den venösen Ablauf 202b gepumpt wird. In der Adsorptionseinrichtung 207 befindet sich ein Adsorberbett, wobei die Adsorberpartikel eine mittlere Partikelgröße von 120 µm und eine mittlere Porengröße von 15 bis 20 nm aufweisen (Adsorber #1997 - vgl. Bsp. 4).

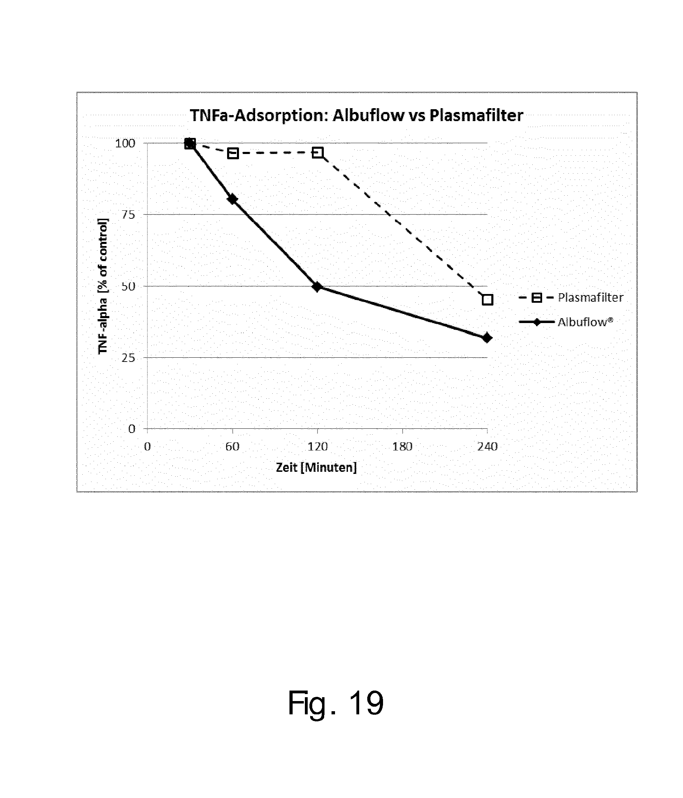

Die Fig. 19 zeigt die Adsorption des Zytokins TNF-α (in [%] in Bezug auf eine Kontrolle ohne Adsorber) im Zeitverlauf unter Verwendung eines Systemaufbaus wie in Fig. 18 gezeigt, wobei jedoch anstelle eines Patienten eine in einem Gefäß vorgelegte Blutprobe (entsprechend dem Gefäß 101 der Fig. 11) verwendet wurde. Dabei wurde ein Albuflow®-Filter (Hersteller: Fresenius Medical Care; Material: Polysulfon-Hohlfasern; Siebkoeffizient für Albumin von ≥ 0,6 und für Fibrinogen ≤ 0,1), der fraktioniertes Plasma durchlässt, mit einem herkömmlichen Plasmafilter, der nur die zellulären Blutbestandteile zurückhält, verglichen. Die Ergebnisse zeigen, dass die Adsorptionsleistung der Adsorptionseinrichtung (befüllt mit Adsorberpartikeln mit einer mittleren Partikelgröße von 120 µm und einer mittleren Porengrößen von 15-20 nm (Adsorber #1997 - vgl. Bsp. 4) für TNF-α bei Anwendung eines Albuflow® Filters deutlich besser ist als mit einem herkömmlichen Plasmafilter. Es kann somit festgestellt werden, dass ein Sorptionsmittel gemäß der Erfindung vor allem in fraktioniertem Plasma hocheffizient arbeitet, und weniger effizient in Plasma oder Vollblut.

### 6. BEISPIEL 6: Extrakorporale Perfusionsvorrichtung mit einem geschlossenen Filtratkreislauf

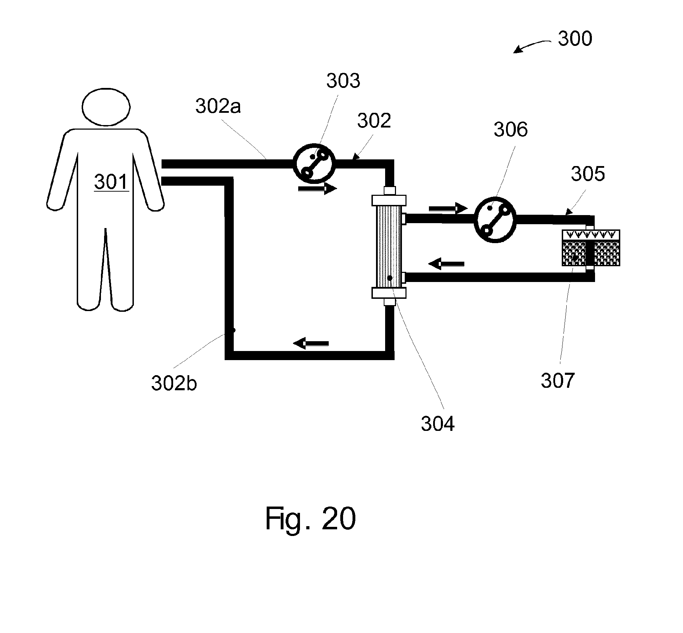

Die Fig. 20 zeigt eine schematische Darstellung einer extrakorporalen Perfusionsvorrichtung 300 (extrakorporale Blutreinigungsvorrichtung 300). Die Perfusionsvorrichtung 300 weist einen extrakorporalen Blutkreislauf 302 mit einem arteriellen Zulauf 302a (arterieller Schenkel) von einem Patienten 301 zu einem Filter 304 und einen venösen Ablauf 302b (venöser Schenkel) vom Filter 304 zum Patienten 301 auf. Das Patientenblut wird mittels einer Blutpumpe 303 (Pumprate Q_{Blut} = 200 ml/min) im Blutkreislauf 302 geführt. Der Filter 304 hat einen Siebkoeffizienten von 5% für Substanzen mit einer relativen Molmasse von 340 kDa und ist beispielsweise ein Filter des Typs Albuflow® (Hersteller: Fresenius Medical Care; Material: Polysulfon-Hohlfasern; Siebkoeffizient für Albumin von ≥ 0,6 und für Fibrinogen ≤ 0,1). Durch den Plasmafilter 304 wird ein Teil des Blutplasmas (= fraktioniertes Plasma) abfiltriert und einem Filtratkreislauf 305 zugeführt. Ein Filter mit einem Siebkoeffizienten von 5% für Substanzen mit einer relativen Molmasse von 340 kDa lässt fraktioniertes Plasma durch, so dass hochmolekulare Blutplasmabestandteile wie Fibrinogen, Immunglobuline, LDL, HDL etc. zurückgehalten werden, während kleinere Blutbestandteile wie Albumin oder Protein C die Filtermembran passieren. Der Filtratkreislauf 305 ist als ein im Filtratbereich geschlossener Kreislauf realisiert, wobei das im Filtratkreislauf 305 geführte fraktionierte Blutplasma mittels einer Filtratpumpe 306 (Pumprate Q_{frakt. Plasma} = 300 ml/min) durch eine mit erfindungsgemäßen Adsorberpartikeln befüllte Adsorptionseinrichtung 307 geführt wird. In der Adsorptionseinrichtung 307 befindet sich ein Adsorberbett, wobei die Adsorberpartikel eine mittlere Partikelgröße von 120 µm und eine mittlere Porengröße von 15 bis 20 nm aufweisen (Adsorber #1997 - vgl. Bsp. 4).

## Patentansprüche

1. Sorptionsmittel umfassend eine poröse Sorptionsmatrix mit einer neutralen, hydrophoben Oberfläche, einer mittleren Porengröße von 11 bis 20 nm und einer mittleren Partikelgröße von 75 bis 150 µm, zur Behandlung von Erkrankungen und Zuständen, die mit einer erhöhten Konzentration wenigstens eines Entzündungsmediators, insbesondere Zytokins, assoziiert sind.

2. Sorptionsmittel nach Anspruch 1, **dadurch gekennzeichnet, dass** die Sorptionsmatrix eine mittlere Partikelgröße von 100 bis 150 µm aufweist.

3. Sorptionsmittel nach Anspruch 2, **dadurch gekennzeichnet, dass** die Sorptionsmatrix eine mittlere Partikelgröße von 110 bis 130 µm aufweist.

4. Sorptionsmittel nach Anspruch 3, **dadurch gekennzeichnet, dass** die Sorptionsmatrix eine mittlere Partikelgröße von 120 µm aufweist.

5. Sorptionsmittel nach einem der Ansprüche 1 bis 4, **dadurch gekennzeichnet, dass** die Sorptionsmatrix ein neutrales, vorzugsweise synthetisches, Polymer ist.

6. Sorptionsmittel nach Anspruch 5, **dadurch gekennzeichnet, dass** das Polymer ein vernetztes Polystyrol-Polymer oder ein vernetztes Ethyldivinylbenzol-Polymer ist.

7. Sorptionsmittel nach Anspruch 6, **dadurch gekennzeichnet, dass** das Polymer ein Polystyrol-Divinylbenzol-Copolymeroder oder ein Ethylvinylbenzol-Divinylbenzol-Copolymer ist.

8. Sorptionsmittel nach einem der Ansprüche 1 bis 7, **dadurch gekennzeichnet, dass** die Oberfläche der Sorptionsmatrix mit einem endotoxinbindenden Lipopeptid, insbesondere Polymyxin, beschichtet ist.

9. Sorptionsmittel nach einem der Ansprüche 1 bis 8, **dadurch gekennzeichnet, dass** die Erkrankung bzw. der Zustand, der mit einer erhöhten Konzentration wenigstens eines Entzündungsmediators, insbesondere Zytokins, assoziiert ist, ausgewählt ist aus der Gruppe bestehend aus Sepsis, insbesondere septischem Schock, Sepsis-ähnlichen Zuständen, alkoholinduzierter Hepatitis und Autoimmunerkrankungen, insbesondere rheumatoider Arthritis, chronisch-entzündlicher Darmerkrankungen und Psoriasis.

10. Sorptionsmittel nach Anspruch 9 zur Behandlung einer Sepsis, insbesondere septischem Schock.

11. Sorptionsmittel nach Anspruch 9, **dadurch gekennzeichnet, dass** der Sepsis-ähnliche Zustand ausgewählt ist aus der Gruppe bestehend aus akutem Leberversagen und akuter Dekompensation bei chronischem Leberversagen.

12. Sorptionsmittel nach Anspruch 11 zur Behandlung von akutem Leberversagen.

13. Sorptionsmittel nach Anspruch 11 zur Behandlung von akuter Dekompensation bei chronischem Leberversagen.

14. Sorptionseinrichtung (107, 207, 307), enthaltend wenigstens ein Sorptionsmittel nach einem der Ansprüche 1 bis 13.

15. Extrakorporale Perfusionsvorrichtung (100, 200, 300), umfassend wenigstens ein Sorptionsmittel nach einem der Ansprüche 1 bis 13 bzw. wenigstens eine Sorptionseinrichtung (107, 207, 307) nach Anspruch 14.

16. Extrakorporale Perfusionsvorrichtung nach Anspruch 15, umfassend einen extrakorporalen Blutkreislauf (202, 302) und einen Filtratkreislauf (205, 305), der mit dem extrakorporalen Blutkreislauf (202, 302) mittels eines Filters (204, 304) in Verbindung steht, wobei der Filter (204, 304) einen Siebkoeffizienten von 5% für Substanzen mit einer relativen Molmasse von 340 kDa aufweist, und wobei die Sorptionseinrichtung (207, 307) im Filtratkreislauf (205, 305) angeordnet ist.
